# EUROPEAN PATENT SPECIFICATION

(11) **EP 3 402 584 B1**
(45) Date of publication and mention of the grant of the patent: **17.08.2022**
(21) Application number: 17701965.0
(22) Date of filing: 12.01.2017
(51) Int. Cl.: B01D 39/16, A47L 13/16, A47L 13/20, A61F 13/51, D01F 8/04

(54) **NONWOVEN CLEANING SUBSTRATE**
REINIGUNGSSUBSTRAT AUS VLIESSTOFF
SUBSTRAT DE NETTOYAGE NON-TISSÉ

(30) Priority: 12.01.2016 US 201662277950 P
(43) Date of publication of application: 21.11.2018
(73) Proprietor: Georgia-Pacific Mt. Holly LLC, Atlanta, GA 30303 (US)
(72) Inventor: DUTKIEWICZ, Jacek K., Cordova, Tennessee 38016 (US); FONG, Brian, Lakeland, Tennessee 38002 (US); LYMAN, Arinne, Greenville, Wisconsin 54942 (US)
(74) Representative: Grünecker Patent- und Rechtsanwälte PartG mbB
(86) International application number: PCT/US2017/013159
(87) International publication number: WO 2017/123734

(56) References cited:
- EP-A1- 1 350 869
- WO-A1-2014/145804
- WO-A2-2007/084953

## Description

### 2. FIELD OF THE INVENTION

The presently disclosed subject matter relates to new nonwoven materials and their use in cleaning articles. More particularly, the presently disclosed subject matter relates to layered structures that contain a tacky additive.

### 3. BACKGROUND OF THE INVENTION

Nonwoven materials are important in a wide range of cleaning articles, including cleaning wipes, cloths, and sheets. Nonwoven materials made from synthetic and cellulose fibers are suitable for cleaning applications because they can be a disposable and cost-effective single-use alternative to fabric cloths and sponges. Cleaning wipes made from nonwoven materials are used in a broad range of applications, including household, personal care, and industrial applications. For cleaning purposes, it can be desirable to have a durable nonwoven material that does not disintegrate upon use. Additionally, nonwoven materials are flexible and can conform to the surface being cleaned. In certain applications, a nonwoven material can be affixed to a tool, such as a mop or duster, to increase its reach and versatility for cleaning.

For use in cleaning articles, it can be advantageous to have a nonwoven material that can attract and collect particles, such as dirt, crumbs, and dust. Although dry wipes may have electrostatic properties to assist in attracting and collecting such particles, it can be desirable to treat the nonwoven materials to improve these capabilities. For example, the nonwoven material may be treated with a cleaning solution or adhesive to collect and retain particles. However, the solution or adhesive can transfer from the nonwoven material to the surface being cleaned, and leave behind a residue. This can cause damage to the surface or be annoying to consumers. Furthermore, the nonwoven material may only collect a portion of the particles on the surface, necessitating the use of multiple wipes to fully clean a surface.

WO 2007/084953 discloses an allergen trap. The allergen trap includes a woven or nonwoven substrate having at least one strata. The trap is impregnated with or otherwise treated with a tacky adhesive by which allergens may be trapped. An example of an allergen is a dust mite. The tacky adhesive, in turn, may be treated with a miticide or activated carbon.

EP 1350869 discloses a binder fiber containing a metallocene catalyzed polyethylene (mPE) and an adhesion promoter. A web comprising the binder fiber and absorbent is also disclosed. A binder fiber containing polyolefin, an adhesion promoter, and an enhancement agent is further disclosed. The polyolefin may be polypropylene, high density polyethylene, medium density polyethylene, low density polyethylene, linear low density polyethylene, or ultra low density polyethylene, manufactured with either Ziegler-Natta or metallocene catalysts. A web comprising this binder fiber and absorbent is also contemplated. The adhesion promoter may be maleic anhydride grafted polyolefins, or ethylene-acrylic copolymers, or a combination of these. The enhancement agent may be one or more of titanium dioxide, talc, silica, alum (aluminum sulfate), calcium carbonate, and magnesium oxide.

WO 2014/145804 dislcoses a multilayer nonwoven material including two, three, or four layers. The multilayered structure can include a first layer comprising continuous filaments and a second layer comprising bonded fibers. The continuous filaments can be synthetic filaments. The fibers can be cellulosic fibers, noncellulosic fibers, or combinations thereof. Certain layers can also contain a binder material.

There remains a need for a durable nonwoven material for cleaning and collecting particles from a surface. The disclosed subject matter addresses these needs.

### 4. SUMMARY

The presently disclosed subject matter provides for a multi-layer nonwoven material comprising at least two layers, at least three layers, at least four layers, or at least five layers, where each of the layers has a specific layered construction. In certain embodiments, the nonwoven material can be treated with a tacky additive.

In certain embodiments, the disclosed subject matter provides for a multi-layer nonwoven material having a first outer layer containing synthetic fibers and a tacky additive, and a second outer layer comprising cellulose fibers and/or synthetic fibers. When used to clean a surface, the nonwoven material has a cleaning efficiency of at least about 20%.

In certain embodiments, the nonwoven material can have a cleaning efficiency of at least about 35%, or at least about 50%. In certain embodiments, the nonwoven material can have a cleaning efficiency of at least 60% with respect to particles having an average particle size of from about 0.4 mm to about 1.0 mm, or from about 0.5 mm to about 0.6 mm. In certain embodiments, the nonwoven material can have a tack of at least about 65 g, at least about 120 g, or at least about 790 g. In certain embodiments, the nonwoven material can have a maximum load of at least about 0.68 N (0.15 lbf), at least about 1.11 N (0.25 1bf), or at least about 7.78 N (1.75 lbf).

In particular embodiments, the synthetic fibers of the first outer layer are bicomponent fibers. The second outer layer can include synthetic fibers. The tacky additive can be a pressure sensitive adhesive. The tacky additive can be present on at least a portion of an external surface of the first outer layer. In certain embodiments, the tacky additive can be present in an amount of from about 0.5 gsm to about 10 gsm, or from about 7 gsm to about 8 gsm, or from about 0.5 gsm to about 1.5 gsm. In certain embodiments, the first outer layer and/or the second outer layer can include binder. The first outer layer can include both bicomponent fibers and cellulose fibers. The second outer layer can include both cellulose fibers and synthetic fibers.

In particular embodiments, the multi-layer nonwoven material can further have an intermediate layer containing bicomponent fibers. In certain embodiments, the multi-layer nonwoven material can be used in a cleaning wipe or a mop head.

In certain embodiments, the disclosed subject matter provides for a multi-layer nonwoven material having an outer layer containing synthetic fibers and a tacky additive, and an absorbent core, which when used to clean a surface, the nonwoven material has a cleaning efficiency of at least about 20%. The absorbent core can include a first layer containing cellulose fibers, a second layer containing SAP, a third layer containing cellulose fibers, a fourth layer containing SAP, and a fifth layer containing cellulose fibers.

In certain embodiments, the nonwoven material can have a cleaning efficiency of at least about 35%, or at least about 50%. In certain embodiments, the nonwoven material can have a cleaning efficiency of at least 60% with respect to particles having an average particle size of from about 0.4 mm to about 1.0 mm, or from about 0.5 mm to about 0.6 mm. In certain embodiments, the nonwoven material can have a tack of at least about 65 g or at least about 790 g. In certain embodiments, the nonwoven material can have a maximum load of at least about 0.68 N (0.15 1bf) or at least about 7.78N (1.75 lbf).

In certain embodiments, the tacky additive can be a pressure sensitive adhesive. The tacky additive can be present on at least a portion of an external surface of the first outer layer. In certain embodiments, the tacky additive can be present in an amount of from about 0.5 gsm to about 10 gsm, or from about 7 gsm to about 8 gsm, or from about 0.5 gsm to about 1.5 gsm.

In certain embodiments, the multi-layer nonwoven material can be used in a cleaning wipe or a mop head.

### 5. BRIEF DESCRIPTION OF THE DRAWINGS

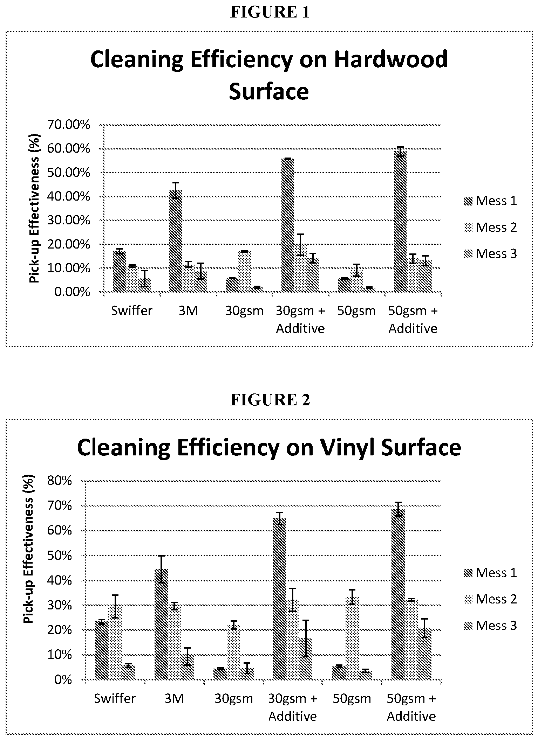

Figure 1 provides an illustration of the cleaning efficiency (%) of the nonwoven materials of Example 1 when tested on a hardwood surface. The nonwoven materials were a 30 gsm material with and without a tacky additive and a 50 gsm material with and without a tacky additive. Cleaning efficiency was tested using three different mess formulations, and is referred to as "Pick-Up Effectiveness (%)" in Figure 1. The cleaning efficiencies of two commercially available products (Swiffer and 3M) are provided in Figure 1 for comparison.
Figure 2 provides an illustration of the cleaning efficiency of the nonwoven materials of Example 1 when tested on a vinyl surface. The nonwoven materials were a 30 gsm material with and without a tacky additive and a 50 gsm material with and without a tacky additive. Cleaning efficiency was tested using three different mess formulations, and is referred to as "Pick-Up Effectiveness (%)" in Figure 2. The cleaning efficiencies of two commercially available products (Swiffer and 3M) are provided in Figure 2 for comparison.

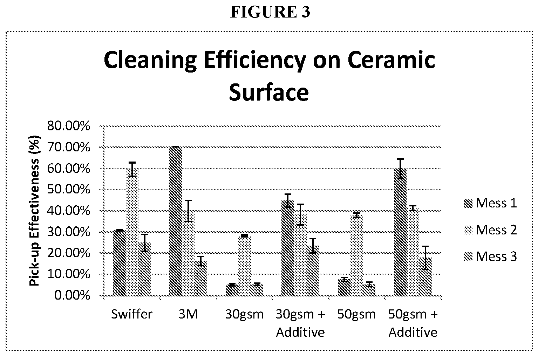

Figure 3 provides an illustration of the cleaning efficiency of the nonwoven materials of Example 1 when tested on a ceramic surface. The nonwoven materials were a 30 gsm material with and without a tacky additive and a 50 gsm material with and without a tacky additive. Cleaning efficiency was tested using three different mess formulations, and is referred to as "Pick-Up Effectiveness (%)" in Figure 3. The cleaning efficiencies of two commercially available products (Swiffer and 3M) are provided in Figure 3 for comparison.

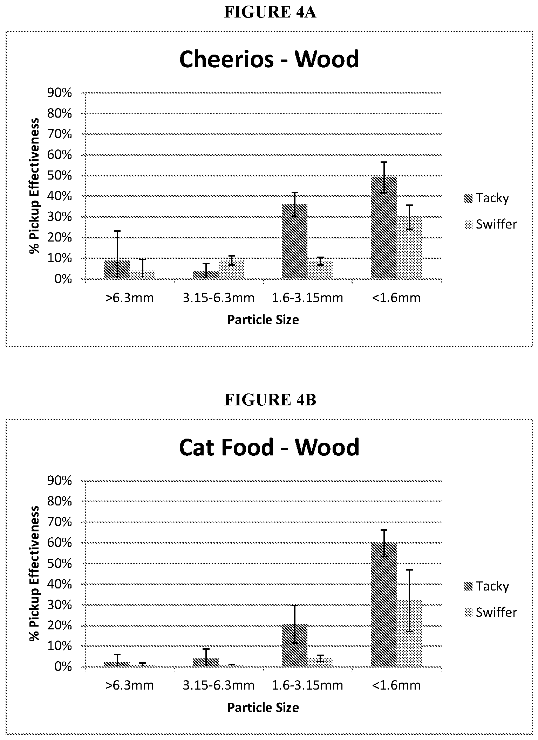

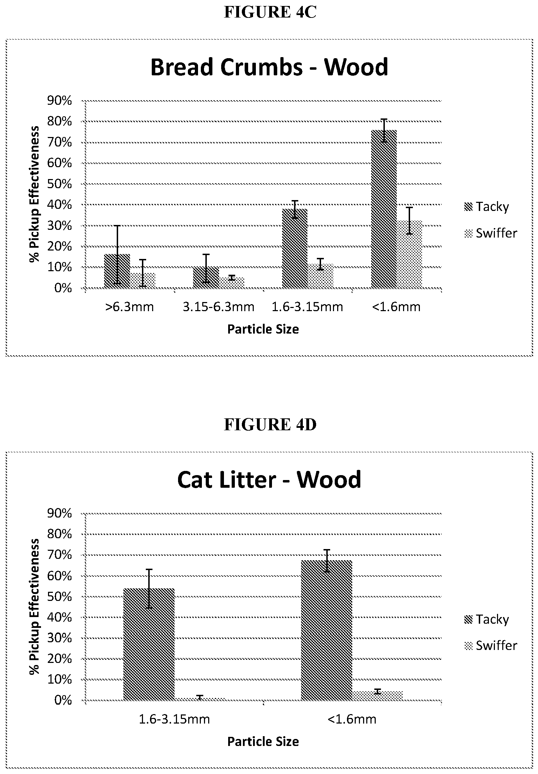

Figures 4A-4D provide an illustration of the cleaning efficiency of the nonwoven materials of Example 2 when tested on a hardwood surface. Each of Figures 4A-4D represents a different mess formulation. The mess formulations were separated by particle size and cleaning efficiency was tested for each particle size. The cleaning efficiency of a commercially available product (Swiffer) is provided in Figures 4A-4D for comparison.

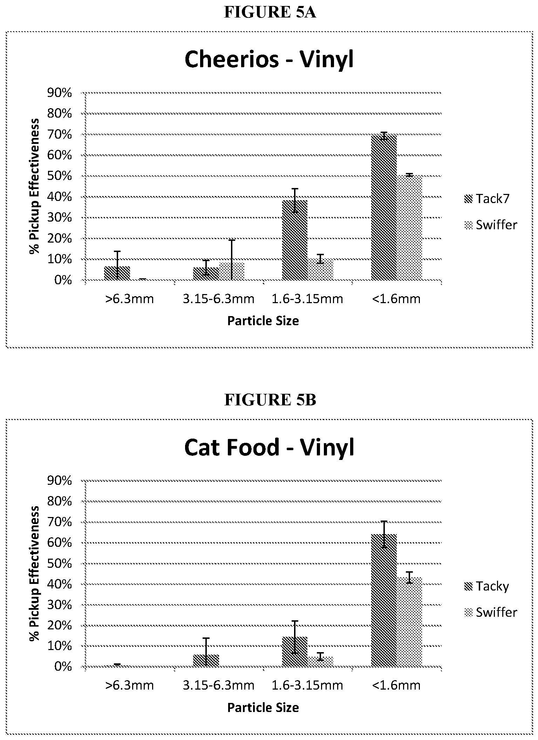

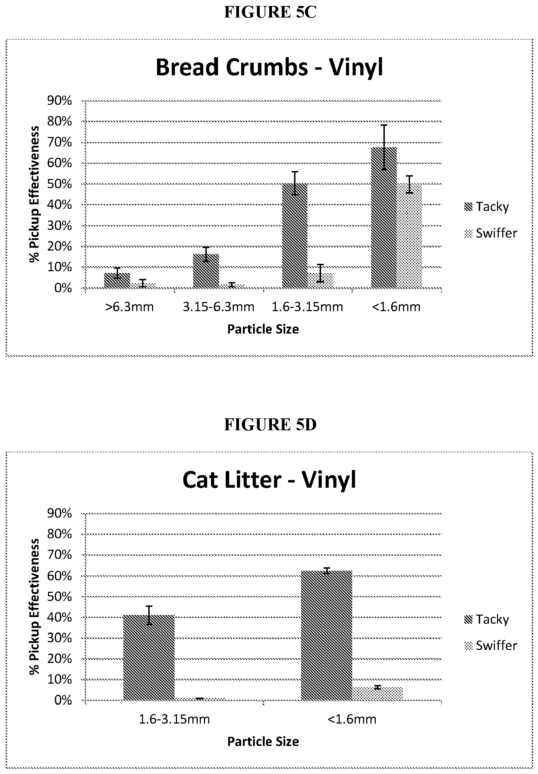

Figures 5A-5D provide an illustration of the cleaning efficiency of the nonwoven materials of Example 2 when tested on a vinyl surface. Each of Figures 5A-5D represents a different mess formulation. The mess formulations were separated by particle size and cleaning efficiency was tested for each particle size. The cleaning efficiency of a commercially available product (Swiffer) is provided in Figures 5A-5D for comparison.

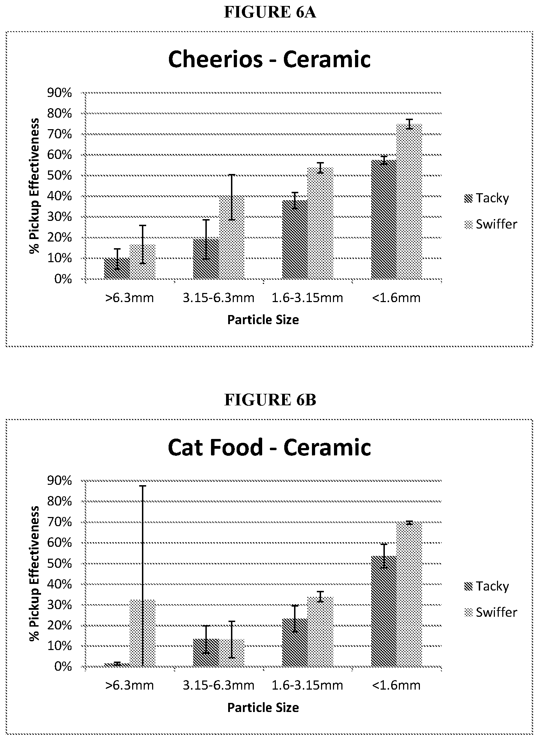

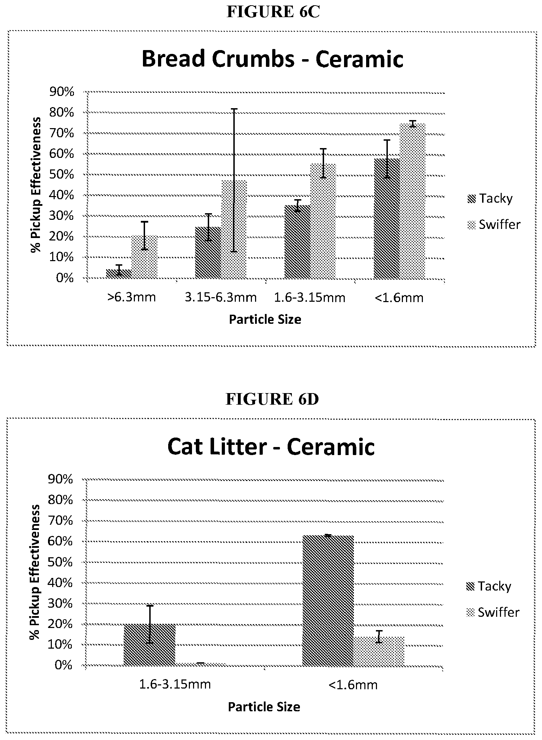

Figures 6A-6D provide an illustration of the cleaning efficiency of the nonwoven materials of Example 2 when tested on a ceramic surface. Each of Figures 6A-6D represents a different mess formulation. The mess formulations were separated by particle size and cleaning efficiency was tested for each particle size. The cleaning efficiency of a commercially available product (Swiffer) is provided in Figures 6A-6D for comparison.

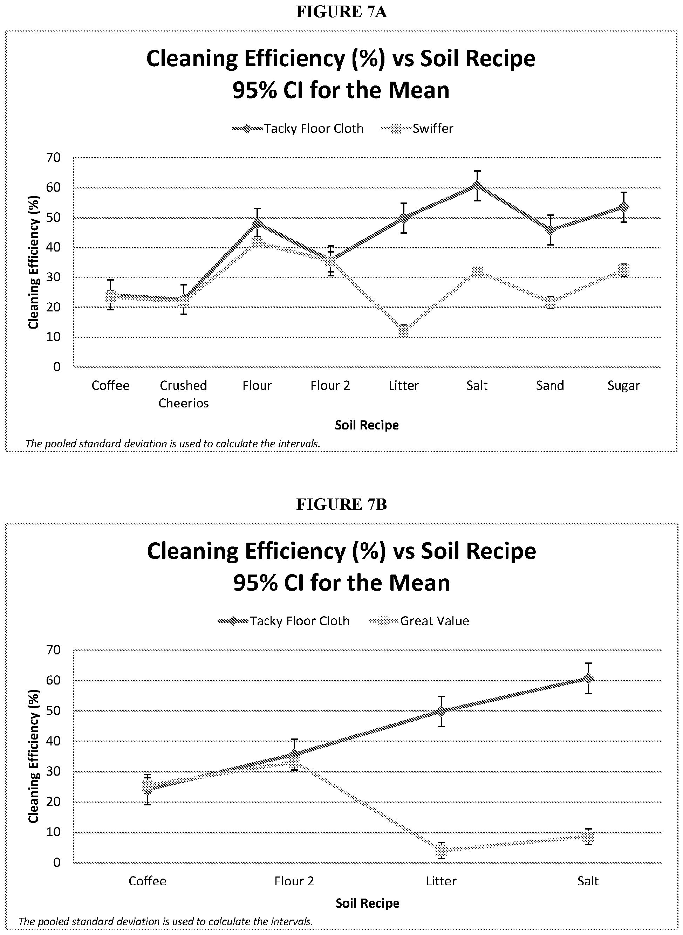

Figures 7A-7B provide the cleaning efficiency of the nonwoven material of Example 3 (*i.e.,* Sample 3) and the commercially available products of Example 3, when used to clean various soil types. Figure 7A compares the cleaning efficiencies of Sample 3 and a commercially available Swiffer product. Figure 7B compares the cleaning efficiencies of Sample 3 and a commercially available Great Value product.

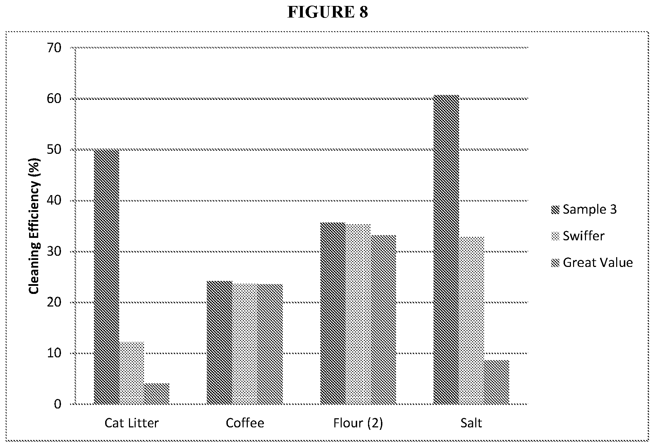

Figure 8 compares the cleaning efficiencies of Sample 3 and the commercially available Swiffer and Great Value products for three soil types: coffee, cat litter, flour, and salt.

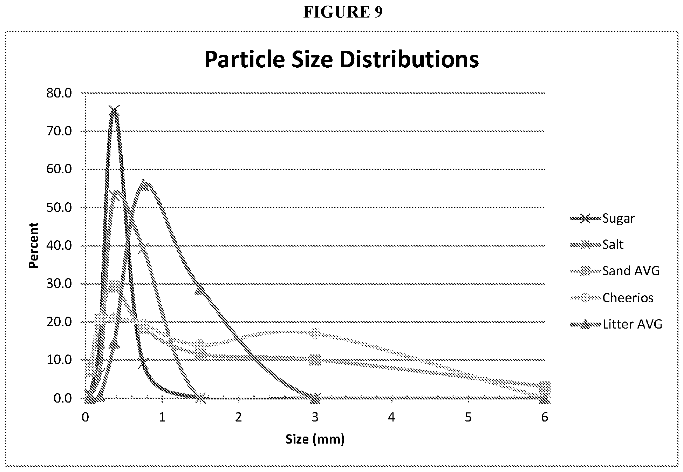

Figure 9 provides particle size distributions for sugar, salt, sand, crushed Cheerios, and cat litter.

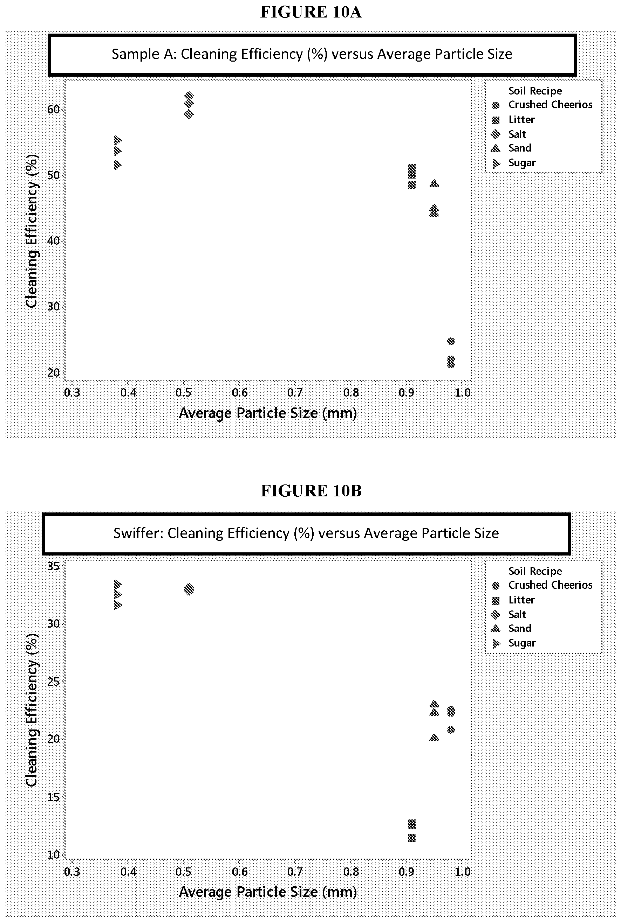

Figures 10A-10B illustrate cleaning efficiency as compared to average particle size for several soil types. Figure 10A provides the cleaning efficiency of the nonwoven material of Example 3 (*i.e.,* Sample 3) for soil types having various particle sizes. Figure 10B provides the cleaning efficiency of a commercially available Swiffer product for soil types having various particle sizes.

### 6. DETAILED DESCRIPTION

The presently disclosed subject matter provides a multi-layer nonwoven material with a tacky additive having at least two layers, and methods for making such materials. These and other aspects of the disclosed subject matter are discussed more in the detailed description and examples.

### Definitions

The terms used in this specification generally have their ordinary meanings in the art, within the context of this subject matter and in the specific context where each term is used. Certain terms are defined below to provide additional guidance in describing the compositions and methods of the disclosed subject matter and how to make and use them.

As used herein, a "nonwoven" refers to a class of material, including but not limited to textiles or plastics. Nonwovens are sheet or web structures made of fiber, filaments, molten plastic, or plastic films bonded together mechanically, thermally, or chemically. A nonwoven is a fabric made directly from a web of fiber, without the yarn preparation necessary for weaving or knitting. In a nonwoven, the assembly of fibers is held together by one or more of the following: (1) by mechanical interlocking in a random web or mat; (2) by fusing of the fibers, as in the case of thermoplastic fibers; or (3) by bonding with a cementing medium such as a natural or synthetic resin.

As used herein, the term "tacky additive" means an additive that has a residual tacky, sticky, or adhesive quality. The tacky additives of the presently disclosed subject matter remain tacky over time. In certain embodiments, the tacky additive can be a material that is permanently tacky at room temperature. Non-limiting examples of tacky additives include adhesives, tackifiers, paints, inks, and any other substance that retains a tacky quality in its dry form. Further non-limiting examples of tacky additives are described in U.S. Patent Publication No. 2010/0095846, the disclosure of which is hereby incorporated by reference in its entirety.

As used herein, the term "weight percent" is meant to refer to either (i) the quantity by weight of a constituent/component in the material as a percentage of the weight of a layer of the material; or (ii) to the quantity by weight of a constituent/component in the material as a percentage of the weight of the final nonwoven material or product.

The term "basis weight" as used herein refers to the quantity by weight of a compound over a given area. Examples of the units of measure include grams per square meter as identified by the acronym "gsm".

As used herein, the term "cleaning efficiency" refers to the percentage of a mess removed by a material, when compared to the original amount of mess present. For example, cleaning efficiency can be calculated by determining the percentage of a known amount of mess that is picked up by a material upon cleaning the mess using the material.

As used herein, the phrase "particle size" or "average particle size" refers to the size of particles, e.g., in a mess formulation. Particle size can be based on a diameter of the particles.

As used in the specification and the appended claims, the singular forms "a," "an" and "the" include plural referents unless the context clearly dictates otherwise. Thus, for example, reference to "a compound" includes mixtures of compounds.

The term "about" or "approximately" means within an acceptable error range for the particular value as determined by one of ordinary skill in the art, which will depend in part on how the value is measured or determined, *i.e.,* the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to systems or processes, the term can mean within an order of magnitude, preferably within 5-fold, and more preferably within 2-fold, of a value.

### Fibers

The nonwoven material of the presently disclosed subject matter comprises one or more types of fibers. For example, the fibers can be natural, synthetic, or a mixture thereof. In certain embodiments, the nonwoven material can contain two or more layers, where each layer contains a specific fibrous content, which can include one or more of synthetic fibers, cellulose-based fibers, or a mixture thereof.

### Synthetic Fibers

In certain embodiments, the nonwoven material can include one or more synthetic layers. Any synthetic fibers known in the art can be used in a synthetic layer. In one embodiment, the synthetic fibers comprise bicomponent and/or mono-component fibers. Bicomponent fibers having a core and sheath are known in the art. Many varieties are used in the manufacture of nonwoven materials, particularly those produced for use in airlaid techniques. Various bicomponent fibers suitable for use in the presently disclosed subject matter are disclosed in U.S. Patent Nos. 5,372,885 and 5,456,982, both of which are hereby incorporated by reference in their entireties. Examples of bicomponent fiber manufacturers include, but are not limited to, Trevira (Bobingen, Germany), Fiber Innovation Technologies (Johnson City, TN) and ES Fiber Visions (Athens, GA).

Bicomponent fibers can incorporate a variety of polymers as their core and sheath components. Bicomponent fibers that have a PE (polyethylene) or modified PE sheath typically have a PET (polyethylene terephthalate) or PP (polypropylene) core. In one embodiment, the bicomponent fiber has a core made of polypropylene and sheath made of polyethylene.

The denier of the bicomponent fiber preferably ranges from about 1.0 dpf to about 4.0 dpf, and more preferably from about 1.5 dpf to about 2.5 dpf. The length of the bicomponent fiber can be from about 3 mm to about 36 mm, preferably from about 3 mm to about 12 mm, more preferably from about 3 mm to about 10 mm. In particular embodiments, the length of the bicomponent fiber is from about 2 mm to about 8 mm, or about 4 mm, or about 6 mm.

Bicomponent fibers are typically fabricated commercially by melt spinning. In this procedure, each molten polymer is extruded through a die, for example, a spinneret, with subsequent pulling of the molten polymer to move it away from the face of the spinneret. This is followed by solidification of the polymer by heat transfer to a surrounding fluid medium, for example chilled air, and taking up of the now solid filament. Non-limiting examples of additional steps after melt spinning can also include hot or cold drawing, heat treating, crimping and cutting. This overall manufacturing process is generally carried out as a discontinuous two-step process that first involves spinning of the filaments and their collection into a tow that comprises numerous filaments. During the spinning step, when molten polymer is pulled away from the face of the spinneret, some drawing of the filament does occur which can also be called the draw-down. This is followed by a second step where the spun fibers are drawn or stretched to increase molecular alignment and crystallinity and to give enhanced strength and other physical properties to the individual filaments. Subsequent steps can include, but are not limited to, heat setting, crimping and cutting of the filament into fibers. The drawing or stretching step can involve drawing the core of the bicomponent fiber, the sheath of the bicomponent fiber or both the core and the sheath of the bicomponent fiber depending on the materials from which the core and sheath are comprised as well as the conditions employed during the drawing or stretching process.

Bicomponent fibers can also be formed in a continuous process where the spinning and drawing are done in a continuous process. During the fiber manufacturing process it is desirable to add various materials to the fiber after the melt spinning step at various subsequent steps in the process. These materials can be referred to as "finish" and be comprised of active agents such as, but not limited to, lubricants and anti-static agents. The finish is typically delivered via an aqueous based solution or emulsion. Finishes can provide desirable properties for both the manufacturing of the bicomponent fiber and for the user of the fiber, for example in an airlaid or wetlaid process.

Numerous other processes are involved before, during and after the spinning and drawing steps and are disclosed in U.S. Patent Nos. 4,950,541, 5,082,899, 5,126,199, 5,372,885, 5,456,982, 5,705,565, 2,861,319, 2,931,091, 2,989,798, 3,038,235, 3,081,490, 3,117,362, 3,121,254, 3,188,689, 3,237,245, 3,249,669, 3,457,342, 3,466,703, 3,469,279, 3,500,498, 3,585,685, 3,163,170, 3,692,423, 3,716,317, 3,778,208, 3,787,162, 3,814,561, 3,963,406, 3,992,499, 4,052,146, 4,251,200, 4,350,006, 4,370,114, 4,406,850, 4,445,833, 4,717,325, 4,743,189, 5,162,074, 5,256,050, 5,505,889, 5,582,913, and 6,670,035, all of which are hereby incorporated by reference in their entireties.

The presently disclosed subject matter can also include, but are not limited to, articles that contain bicomponent fibers that are partially drawn with varying degrees of draw or stretch, highly drawn bicomponent fibers and mixtures thereof. These can include, but are not limited to, a highly drawn polyester core bicomponent fiber with a variety of sheath materials, specifically including a polyethylene sheath such as Trevira T255 (Bobingen, Germany) or a highly drawn polypropylene core bicomponent fiber with a variety of sheath materials, specifically including a polyethylene sheath such as ES FiberVisions AL-Adhesion-C (Varde, Denmark). Additionally, Trevira T265 bicomponent fiber (Bobingen, Germany), having a partially drawn core with a core made of polybutylene terephthalate (PBT) and a sheath made of polyethylene can be used. The use of both partially drawn and highly drawn bicomponent fibers in the same structure can be leveraged to meet specific physical and performance properties based on how they are incorporated into the structure.

The bicomponent fibers of the presently disclosed subject matter are not limited in scope to any specific polymers for either the core or the sheath as any partially drawn core bicomponent fiber can provide enhanced performance regarding elongation and strength. The degree to which the partially drawn bicomponent fibers are drawn is not limited in scope as different degrees of drawing will yield different enhancements in performance. The scope of the partially drawn bicomponent fibers encompasses fibers with various core sheath configurations including, but not limited to concentric, eccentric, side by side, islands in a sea, pie segments and other variations. The relative weight percentages of the core and sheath components of the total fiber can be varied. In addition, the scope of this subject matter covers the use of partially drawn homopolymers such as polyester, polypropylene, nylon, and other melt spinnable polymers. The scope of this subject matter also covers multicomponent fibers that can have more than two polymers as part of the fibers structure.

In particular embodiments, the bicomponent fibers in a particular layer comprise from about 10 to about 100 percent by weight of the layer. In alternative embodiments, the bicomponent layer contains from about 10 gsm to about 50 gsm bicomponent fibers, or from about 20 gsm to about 50 gsm bicomponent fibers, or from about 30 gsm to about 40 gsm bicomponent fibers.

In particular embodiments, the bicomponent fibers are low dtex staple bicomponent fibers in the range of about 0.5 dtex to about 20 dtex. In certain embodiments, the dtex value is 5.7 dtex. In other certain embodiments, the dtex value is 1.7 dtex.

Other synthetic fibers suitable for use in various embodiments as fibers or as bicomponent binder fibers include, but are not limited to, fibers made from various polymers including, by way of example and not by limitation, acrylic, polyamides (including, but not limited to, Nylon 6, Nylon 6/6, Nylon 12, polyaspartic acid, polyglutamic acid), polyamines, polyimides, polyacrylics (including, but not limited to, polyacrylamide, polyacrylonitrile, esters of methacrylic acid and acrylic acid), polycarbonates (including, but not limited to, polybisphenol A carbonate, polypropylene carbonate), polydienes (including, but not limited to, polybutadiene, polyisoprene, polynorbomene), polyepoxides, polyesters (including, but not limited to, polyethylene terephthalate, polybutylene terephthalate, polytrimethylene terephthalate, polycaprolactone, polyglycolide, polylactide, polyhydroxybutyrate, polyhydroxyvalerate, polyethylene adipate, polybutylene adipate, polypropylene succinate), polyethers (including, but not limited to, polyethylene glycol (polyethylene oxide), polybutylene glycol, polypropylene oxide, polyoxymethylene (paraformaldehyde), polytetramethylene ether (polytetrahydrofuran), polyepichlorohydrin), polyfluorocarbons, formaldehyde polymers (including, but not limited to, urea-formaldehyde, melamine-formaldehyde, phenol formaldehyde), natural polymers (including, but not limited to, cellulosics, chitosans, lignins, waxes), polyolefins (including, but not limited to, polyethylene, polypropylene, polybutylene, polybutene, polyoctene), polyphenylenes (including, but not limited to, polyphenylene oxide, polyphenylene sulfide, polyphenylene ether sulfone), silicon containing polymers (including, but not limited to, polydimethyl siloxane, polycarbomethyl silane), polyurethanes, polyvinyls (including, but not limited to, polyvinyl butyral, polyvinyl alcohol, esters and ethers of polyvinyl alcohol, polyvinyl acetate, polystyrene, polymethylstyrene, polyvinyl chloride, polyvinyl pryrrolidone, polymethyl vinyl ether, polyethyl vinyl ether, polyvinyl methyl ketone), polyacetals, polyarylates, and copolymers (including, but not limited to, polyethylene-co-vinyl acetate, polyethylene-co-acrylic acid, polybutylene terephthalate-co-polyethylene terephthalate, polylauryllactam-block-polytetrahydrofuran), polybutylene succinate and polylactic acid based polymers.

In particular embodiments, polypropylene fibers are used in a synthetic fiber layer. In specific embodiments, spunbond polypropylene fibers are used in a synthetic fiber layer. In certain embodiments, the synthetic fiber layer contains from about 5 gsm to about 20 gsm synthetic fibers, or about 5 gsm to about 15 gsm synthetic fibers, or from about 8 gsm to about 12 gsm synthetic fibers. In particular embodiments, the synthetic fiber layer contains from about 8 gsm to about 12 gsm synthetic fibers.

### Cellulose Fibers

In addition to the use of synthetic fibers, the presently disclosed subject matter also contemplates the use of cellulose-based fibers, either alone or in combination with synthetic fibers. In certain embodiments, the nonwoven material can include one or more cellulosic layers. Any cellulose fibers known in the art, including cellulose fibers of any natural origin, such as those derived from wood pulp or regenerated cellulose, can be used in a cellulosic layer. In certain embodiment, cellulose fibers include, but are not limited to, digested fibers, such as kraft, prehydrolyzed kraft, soda, sulfite, chemi-thermal mechanical, and thermo-mechanical treated fibers, derived from softwood, hardwood or cotton linters. In other embodiments, cellulose fibers include, but are not limited to, kraft digested fibers, including prehydrolyzed kraft digested fibers. Non-limiting examples of cellulosic fibers suitable for use in this subject matter are the cellulose fibers derived from softwoods, such as pines, firs, and spruces. Other suitable cellulose fibers include, but are not limited to, those derived from Esparto grass, bagasse, kemp, flax, hemp, kenaf, and other lignaceous and cellulosic fiber sources. Suitable cellulose fibers include, but are not limited to, bleached Kraft southern pine fibers sold under the trademark FOLEY FLUFFS^{®} (Buckeye Technologies Inc., Memphis, Tenn.). Additionally, fibers sold under the trademark CELLU TISSUE^{®} (*e.g*., Grade 3024) (Clearwater Paper Corporation, Spokane, Wash.) are utilized in certain aspects of the disclosed subject matter.

The nonwoven materials of the disclosed subject matter can also include, but are not limited to, a commercially available bright fluff pulp including, but not limited to, southern softwood fluff pulp (such as Treated FOLEY FLUFFS^{®}) northern softwood sulfite pulp (such as T 730 from Weyerhaeuser), or hardwood pulp (such as eucalyptus). While certain pulps may be preferred based on a variety of factors, any absorbent fluff pulp or mixtures thereof can be used. In certain embodiments, wood cellulose, cotton linter pulp, chemically modified cellulose such as cross-linked cellulose fibers and highly purified cellulose fibers can be used. Non-limiting examples of additional pulps are FOLEY FLUFFS^{®} FFTAS (also known as FFTAS or Buckeye Technologies FFT-AS pulp), and Weyco CF401.

Other suitable types of cellulose fiber include, but are not limited to, chemically modified cellulose fibers. In particular embodiments, the modified cellulose fibers are crosslinked cellulose fibers. U.S. Patent Nos. 5,492,759; 5,601,921; 6,159,335, all of which are hereby incorporated by reference in their entireties, relate to chemically treated cellulose fibers useful in the practice of this disclosed subject matter. In certain embodiments, the modified cellulose fibers comprise a polyhydroxy compound. Non-limiting examples of polyhydroxy compounds include glycerol, trimethylolpropane, pentaerythritol, polyvinyl alcohol, partially hydrolyzed polyvinyl acetate, and fully hydrolyzed polyvinyl acetate. In certain embodiments, the fiber is treated with a polyvalent cation-containing compound. In one embodiment, the polyvalent cation-containing compound is present in an amount from about 0.1 weight percent to about 20 weight percent based on the dry weight of the untreated fiber. In particular embodiments, the polyvalent cation containing compound is a polyvalent metal ion salt. In certain embodiments, the polyvalent cation containing compound is selected from the group consisting of aluminum, iron, tin, salts thereof, and mixtures thereof. Any polyvalent metal salt including transition metal salts may be used. Non-limiting examples of suitable polyvalent metals include beryllium, magnesium, calcium, strontium, barium, titanium, zirconium, vanadium, chromium, molybdenum, tungsten, manganese, iron, cobalt, nickel, copper, zinc, aluminum and tin. Preferred ions include aluminum, iron and tin. The preferred metal ions have oxidation states of +3 or +4. Any salt containing the polyvalent metal ion may be employed. Non-limiting examples of suitable inorganic salts of the above metals include chlorides, nitrates, sulfates, borates, bromides, iodides, fluorides, nitrides, perchlorates, phosphates, hydroxides, sulfides, carbonates, bicarbonates, oxides, alkoxides phenoxides, phosphites, and hypophosphites. Non-limiting examples of suitable organic salts of the above metals include formates, acetates, butyrates, hexanoates, adipates, citrates, lactates, oxalates, propionates, salicylates, glycinates, tartrates, glycolates, sulfonates, phosphonates, glutamates, octanoates, benzoates, gluconates, maleates, succinates, and 4,5-dihydroxy-benzene-1,3-disulfonates. In addition to the polyvalent metal salts, other compounds such as complexes of the above salts include, but are not limited to, amines, ethylenediaminetetra-acetic acid (EDTA), diethylenetriaminepenta-acetic acid (DIPA), nitrilotri-acetic acid (NTA), 2,4-pentanedione, and ammonia may be used.

In one embodiment, the cellulose pulp fibers are chemically modified cellulose pulp fibers that have been softened or plasticized to be inherently more compressible than unmodified pulp fibers. The same pressure applied to a plasticized pulp web will result in higher density than when applied to an unmodified pulp web. Additionally, the densified web of plasticized cellulose fibers is inherently softer than a similar density web of unmodified fiber of the same wood type. Softwood pulps may be made more compressible using cationic surfactants as debonders to disrupt interfiber associations. Use of one or more debonders facilitates the disintegration of the pulp sheet into fluff in the airlaid process. Examples of debonders include, but are not limited to, those disclosed in U.S. Patent Nos. 4,432,833, 4,425,186 and 5,776,308, all of which are hereby incorporated by reference in their entireties. One example of a debonder-treated cellulose pulp is FFLE+. Plasticizers for cellulose, which can be added to a pulp slurry prior to forming wetlaid sheets, can also be used to soften pulp, although they act by a different mechanism than debonding agents. Plasticizing agents act within the fiber, at the cellulose molecule, to make flexible or soften amorphous regions. The resulting fibers are characterized as limp. Since the plasticized fibers lack stiffness, the comminuted pulp is easier to densify compared to fibers not treated with plasticizers. Plasticizers include, but are not limited to, polyhydric alcohols such as glycerol, low molecular weight polyglycol such as polyethylene glycols, and polyhydroxy compounds. These and other plasticizers are described and exemplified in U.S. Pat. Nos. 4,098,996, 5,547,541 and 4,731,269, all of which are hereby incorporated by reference in their entireties. Ammonia, urea, and alkylamines are also known to plasticize wood products, which mainly contain cellulose (A. J. Stamm, Forest Products Journal 5(6):413, 1955, hereby incorporated by reference in its entirety).

In particular embodiments of the disclosed subject matter, GP4723, a fully-treated cellulose pulp (available from Georgia-Pacific) is used in a cellulose fiber layer. In particular embodiments, the cellulose fiber layer contains from about 5 gsm to about 100 gsm cellulose fibers, or from about 7 gsm to about 50 gsm, or about 9 gsm to about 30 gsm.

### Additives

The nonwoven material of the presently disclosed subject matter can include one or more additives. For example, in certain embodiments, the nonwoven material can include a tacky additive and/or a binder. The nonwoven material can also include other additives.

### Tacky Additives

In certain embodiments, the nonwoven material can be coated with a tacky additive. Suitable tacky additives can be materials having inherent tacky or sticky qualities. Alternatively, the tacky additive can be a material that is combined with a suitable tackifier to produce tacky qualities. Various tacky additives are described in U.S. Patent Publication No. 2010/0095846, the disclosure of which is incorporated by reference in its entirety.

Suitable tacky additives include adhesives. For example, and not limitation, such adhesives include pressure sensitive adhesives. The pressure sensitive adhesives can be water-based. For example, such adhesives include Cattie 8241E, a high solids, 60-70 wt-% acrylic copolymer emulsion in an aqueous solution.

Other suitable tacky additives can include polymeric paints or inks. For example, certain polymeric paints or inks remain tacky after drying. Such polymeric paints include Polytex paint. Further examples of suitable polymeric paints are described in U.S. Patent Publication No. 2012/0094091, the disclosure of which is incorporated by reference in its entirety.

In certain embodiments, a tacky additive can be applied by spraying, coating, or printing one or more fibrous layers of the nonwoven material. For example, the coverage of the tacky additive on a layer of the nonwoven material can be from about 1% to about 99%. In certain embodiments, the tacky additive can be applied in amounts ranging from about 1 gsm to about 15 gsm, from about 3 gsm to about 12 gsm, or from about 7 gsm to about 8 gsm. A tacky additive can be applied to one side of a fibrous layer, preferably an externally facing layer. Alternatively, a tacky additive can be applied to both sides of a layer, in equal or disproportionate amounts.

### Binders

The nonwoven material of the presently disclosed subject matter can include one or more binders. Suitable binders include, but are not limited to, liquid binders and powder binders. Non-limiting examples of liquid binders include emulsions, solutions, or suspensions of binders. Non-limiting examples of binders include polyethylene powders, copolymer binders, vinylacetate ethylene binders, styrene-butadiene binders, urethanes, urethane-based binders, acrylic binders, thermoplastic binders, natural polymer based binders, and mixtures thereof.

Suitable binders include, but are not limited to, copolymers, vinylacetate ethylene ("VAE") copolymers which can have a stabilizer, such as Wacker Vinnapas 192, Wacker Vinnapas EF 539, Wacker Vinnapas EP907, Wacker Vinnapas EP129, Celanese Duroset E130, Celanese Dur-O-Set Elite 130 25-1813 and Celanese Dur-O-Set TX-849, Celanese 75-524A, polyvinyl alcohol-polyvinyl acetate blends such as Wacker Vinac 911, vinyl acetate homopolyers, polyvinyl amines such as BASF Luredur, acrylics, cationic acrylamides, polyacryliamides such as Bercon Berstrength 5040 and Bercon Berstrength 5150, hydroxyethyl cellulose, starch such as National Starch CATO RTM 232, National Starch CATO RTM 255, National Starch Optibond, National Starch Optipro, or National Starch OptiPLUS, guar gum, styrene-butadienes, urethanes, urethane-based binders, thermoplastic binders, acrylic binders, and carboxymethyl cellulose such as Hercules Aqualon CMC. In certain embodiments, the binder is a natural polymer based binder. Non-limiting examples of natural polymer based binders include polymers derived from starch, cellulose, chitin, and other polysaccharides.

In certain embodiments, the binder is water-soluble. In one embodiment, the binder is a vinylacetate ethylene copolymer. One non-limiting example of such copolymers is EP907 (Wacker Chemicals, Munich, Germany). Vinnapas EP907 can be applied at a level of about 10% solids incorporating about 0.75% by weight Aerosol OT (Cytec Industries, West Paterson, N.J.), which is an anionic surfactant. Other classes of liquid binders such as styrene-butadiene and acrylic binders can also be used.

In certain embodiments, the binder is not water-soluble. Examples of these binders include, but are not limited to, Vinnapas 124 and 192 (Wacker) which can have an opacifier and whitener, including, but not limited to, titanium dioxide, dispersed in the emulsion. Other binders include, but are not limited to, Celanese Emulsions (Bridgewater, N.J.) Elite 22 and Elite 33.

In certain embodiments, the binder is a thermoplastic binder. Such thermoplastic binders include, but are not limited to, any thermoplastic polymer which can be melted at temperatures which will not extensively damage the cellulosic fibers. Preferably, the melting point of the thermoplastic binding material will be less than about 175°C. Examples of suitable thermoplastic materials include, but are not limited to, suspensions of thermoplastic binders and thermoplastic powders. In particular embodiments, the thermoplastic binding material can be, for example, polyethylene, polypropylene, polyvinylchloride, and/or polyvinylidene chloride.

In particular embodiments, the vinylacetate ethylene binder is non-crosslinkable. In one embodiment, the vinylacetate ethylene binder is crosslinkable. In certain embodiments, the binder is WD4047 urethane-based binder solution supplied by HB Fuller. In one embodiment, the binder is Michem Prime 4983-45N dispersion of ethylene acrylic acid ("EAA") copolymer supplied by Michelman. In certain embodiments, the binder is Dur-O-Set Elite 22LV emulsion of VAE binder supplied by Celanese Emulsions (Bridgewater, N.J.). As noted above, in particular embodiments, the binder is crosslinkable. It is also understood that crosslinkable binders are also known as permanent wet strength binders. A permanent wet-strength binder includes, but is not limited to, Kymene^{®} (Hercules Inc., Wilmington, Del.), Parez^{®} (American Cyanamid Company, Wayne, N.J.), Wacker Vinnapas or AF192 (Wacker Chemie AG, Munich, Germany), or the like. Various permanent wet-strength agents are described in U.S. Patent No. 2,345,543, U.S. Patent No. 2,926,116, and U.S. Patent No. 2,926,154, the disclosures of which are incorporated by reference in their entirety. Other permanent wet-strength binders include, but are not limited to, polyamine-epichlorohydrin, polyamide epichlorohydrin or polyamide-amine epichlorohydrin resins, which are collectively termed "PAE resins". Non-limiting exemplary permanent wet-strength binders include Kymene 557H or Kymene 557LX (Hercules Inc., Wilmington, Del.) and have been described in U.S. Patent No. 3,700,623 and U.S. Patent No. 3,772,076, which are incorporated herein in their entirety by reference thereto.

Alternatively, in certain embodiments, the binder is a temporary wet-strength binder. The temporary wet-strength binders include, but are not limited to, Hercobond^{®} (Hercules Inc., Wilmington, Del.), Parez^{®} 750 (American Cyanamid Company, Wayne, N.J.), Parez^{®} 745 (American Cyanamid Company, Wayne, N.J.), or the like. Other suitable temporary wet-strength binders include, but are not limited to, dialdehyde starch, polyethylene imine, mannogalactan gum, glyoxal, and dialdehyde mannogalactan. Other suitable temporary wet-strength agents are described in U.S. Patent No. 3,556,932, U.S. Patent No. 5,466,337, U.S. Patent No. 3,556,933, U.S. Patent No. 4,605,702, U.S. Patent No. 4,603,176, U.S. Patent No. 5,935,383, and U.S. Patent No. 6,017,417, all of which are incorporated herein in their entirety by reference thereto.

In certain embodiments, binders are applied as emulsions in amounts ranging from about 1 gsm to about 4 gsm, or from about 1 gsm to about 2 gsm, or from about 2 gsm to about 3 gsm. Binder can be applied to one side of a fibrous layer, preferably an externally facing layer. Alternatively, binder can be applied to both sides of a layer, in equal or disproportionate amounts.

### Other Additives

The nonwoven materials of the presently disclosed subject matter can also contain other additives. For example, where the nonwoven material includes an absorbent core, one or more layers can contain superabsorbent polymer (SAP). The types of superabsorbent polymers which may be used in the disclosed subject matter include, but are not limited to, SAPs in their particulate form such as powder, irregular granules, spherical particles, staple fibers and other elongated particles. U.S. Patent Nos. 5,147,343; 5,378,528; 5,795,439; 5,807,916; 5,849,211, and 6,403,857, which are hereby incorporated by reference in their entireties, describe various superabsorbent polymers and methods of making superabsorbent polymers. One example of a superabsorbent polymer forming system is crosslinked acrylic copolymers of metal salts of acrylic acid and acrylamide or other monomers such as 2-acrylamido-2-methylpropanesulfonic acid. Many conventional granular superabsorbent polymers are based on poly(acrylic acid) which has been crosslinked during polymerization with any of a number of multi-functional comonomer crosslinking agents well-known in the art. Examples of multi-functional crosslinking agents are set forth in U.S. Patent Nos. 2,929,154; 3,224,986; 3,332,909; 4,076,673, which are hereby incorporated by reference in their entireties. For instance, crosslinked carboxylated polyelectrolytes can be used to form superabsorbent polymers. Other water-soluble polyelectrolyte polymers are known to be useful for the preparation of superabsorbents by crosslinking, these polymers include: carboxymethyl starch, carboxymethyl cellulose, chitosan salts, gelatine salts, etc. They are not, however, commonly used on a commercial scale to enhance absorbency of dispensable absorbent articles mainly due to their higher cost. Superabsorbent polymer granules useful in the practice of this subject matter are commercially available from a number of manufacturers, such as BASF, Dow Chemical (Midland, Mich.), Stockhausen (Greensboro, N.C.), Chemdal (Arlington Heights, Ill.), and Evonik (Essen, Germany). Non-limiting examples of SAP include a surface crosslinked acrylic acid based powder such as Stockhausen 9350 or SX70, BASF HySorb FEM 33N, or Evonik Favor SXM 7900.

In certain embodiments, SAP can be used in a layer in amounts ranging from about 5% to about 50% based on the total weight of the structure. In certain embodiments, the amount of SAP in a layer can range from about 10 gsm to about 50 gsm, or from about 12 gsm to about 40 gsm, or from about 15 gsm to about 25 gsm.

### Nonwoven Materials

The presently disclosed subject matter provides for nonwoven materials. In certain embodiments, a nonwoven material contains at least two layers, wherein each layer comprises a specific fibrous content. In specific embodiments, the nonwoven material contains at least two synthetic fiber layers. In other embodiments, the nonwoven material contains a synthetic fiber layer and a cellulose fiber layer. In certain embodiments, a synthetic fiber layer can include bicomponent fibers.

In certain embodiments, the nonwoven material has at least two layers, wherein each layer comprises a specific fibrous content. In specific embodiments, the first layer is composed of bicomponent fibers. A second layer disposed adjacent to the first layer is composed of synthetic fibers. In particular embodiments, the first layer can further include cellulose fibers in addition to bicomponent fibers. For example, the first layer can include cellulose fluff. In alternative embodiments, the second layer can contain cellulose fibers rather than synthetic fibers. In certain embodiments, the second layer can further include bicomponent fibers in addition to cellulose fibers. In certain embodiments, the second layer is coated with binder on its outer surface.

In certain embodiments, the first layer contains from about 10 gsm to about 50 gsm of bicomponent fibers. In particular embodiments, the bicomponent fibers have an eccentric core sheath configuration. The bicomponent fibers can have a polyethylene sheath and a polypropylene core. In particular embodiments, the first layer further includes from about 5 gsm to about 50 gsm of cellulose fibers. In certain embodiments, the second layer contains from about 5 gsm to about 15 gsm, or from about 8 gsm to about 12 gsm of synthetic fibers. In particular embodiments, the synthetic fibers can include polypropylene. Alternatively, the second layer can contain from about 10 gsm to about 100 gsm of cellulose fibers. The cellulose fibers can be cellulose fluff. In particular embodiments, the second layer can contain from about 5 gsm to about 50 gsm of bicomponent fibers.

In another embodiment, the nonwoven material has at least three layers, wherein each layer has a specific fibrous content. In specific embodiments, the first layer is composed of synthetic fibers. In particular embodiments, the synthetic fibers are bicomponent fibers. A second layer disposed adjacent to the first layer is composed of bicomponent fibers. A third layer disposed adjacent to the second layer is composed of synthetic fibers. In alternative embodiments, the third layer is composed of cellulose fibers. In particular embodiments, the third layer can further include bicomponent fibers in addition to cellulose fibers. In certain embodiments, the third layer is coated with binder on its outer surface.

In certain embodiments, the first layer contains from about 10 gsm to about 50 gsm of bicomponent fibers. In particular embodiments, the bicomponent fibers have an eccentric core sheath configuration. The bicomponent fibers can have a polyethylene sheath and a polypropylene core. In certain embodiments, the second layer contains from about 4 gsm to about 20 gsm of bicomponent fibers. In certain embodiments, the third layer contains from about 5 gsm to about 15 gsm, or from about 8 gsm to about 12 gsm of synthetic fibers. The synthetic fibers can include polypropylene. Alternatively, the third layer can contain from about 10 gsm to about 100 gsm of cellulose fibers. The cellulose fibers can be cellulose fluff. In particular embodiments, the third layer can contain from about 5 gsm to about 50 gsm of bicomponent fibers.

### Absorbent Cores

In certain embodiments, the nonwoven material can include an absorbent core. The inclusion of an absorbent core can increase the overall absorbency of the nonwoven material. For example, a nonwoven material having an absorbent core can be suitable for cleaning wet surfaces or liquid messes. In certain embodiments, the absorbent core can have at least five layers, wherein each layer has a specific fibrous content. In certain embodiments, the first layer contains cellulose fibers, the second layer contains SAP, the third layer contains cellulose fibers, the fourth layer contains SAP, and the fifth layer contains cellulose fibers. In certain embodiments, one or more of the first layer, third layer, and/or fifth layer can further include bicomponent fibers. In certain embodiments, the nonwoven material can further include at least one additional layer adjacent to the absorbent core. In particular embodiments, the additional layer contains synthetic fibers. In certain embodiments, the additional layer can include bicomponent fibers.

In particular embodiments, the first layer contains from about 10 gsm to about 50 gsm of cellulose fibers. In certain embodiments, the second layer contains from about 10 gsm to about 50 gsm of SAP particles. In certain embodiments, the third layer contains from about 10 gsm to about 50 gsm of cellulose fibers. In certain embodiments, the fourth layer contains from about 10 gsm to about 50 gsm of SAP particles. In certain embodiments, the fifth layer contains from about 10 gsm to about 50 gsm of cellulose fibers. In particular embodiments, an additional layer comprises from about 10 gsm to about 50 gsm of synthetic fibers. In particular embodiments, the synthetic fibers are bicomponent fibers having an eccentric core sheath configuration. The bicomponent fibers can have a polyethylene sheath and a polypropylene core.

### Features of the Nonwoven Materials

In certain embodiments, one or more layers are treated with a tacky additive. For example, the outer surfaces of one or more layers can be treated with the tacky additive. In certain embodiments, the tacky additive is applied to a synthetic fiber layer, for example, a bicomponent fiber layer. In certain embodiments, the coverage of the tacky additive on a layer can be from about 1% to about 99%. In particular embodiments, at least a portion of an outer surface is treated with a tacky additive in an amount ranging from about 0.5 gsm to about 20 gsm, from about 1 gsm to about 15 gsm, from about 3 gsm to about 12 gsm, or from about 7 gsm to about 8 gsm. In particular embodiments, at least a portion of an outer surface is treated with a tacky additive in an amount ranging from about 0.5 gsm to about 1.5 gsm.

In certain embodiments, one or more layers are bonded on at least a portion of at least one of their outer surfaces with binder. It is not necessary that the binder chemically bond with a portion of the layer, although it is preferred that the binder remain associated in close proximity with the layer, by coating, adhering, precipitation, or any other mechanism such that it is not dislodged from the layer during normal handling of the layer. For convenience, the association between the layer and the binder discussed above can be referred to as the bond, and the compound can be said to be bonded to the layer. In certain embodiments of the presently disclosed subject matter, at least a portion of at least one outer layer is coated with binder. In particular embodiments of the disclosed subject matter, at least a portion of an outer layer is coated with binder in an amount ranging from about 1 gsm to about 4 gsm, or from about 1 gsm to about 2 gsm, or from about 2 gsm to about 3 gsm.

In certain embodiments of the nonwoven material, the range of the basis weight in a first layer is from about 5 gsm to about 100 gsm, or from about 10 gsm to about 50 gsm. The range of the basis weight in a second layer is from about 5 gsm to about 100 gsm, or from about 5 gsm to about 50 gsm, or from about 5 gsm to about 15 gsm, or from about 8 gsm to about 12 gsm. If additional layers are present, the basis weight of each ranges from about 5 gsm to about 100 gsm, or from about 10 gsm to about 50 gsm, or from about 4 gsm to about 20 gsm.

In certain embodiments of the nonwoven material, the range of basis weight of the overall structure is from about 5 gsm to about 600 gsm, or from about 5 gsm to about 300 gsm, or from about 5 gsm to about 250 gsm, or from about 10 gsm to about 250 gsm, or from about 20 gsm to about 200 gsm, or from about 30 gsm to about 200 gsm, or from about 40 gsm to about 200 gsm.

The caliper of the nonwoven material refers to the caliper of the entire material. In certain embodiments, the caliper of the material ranges from about 0.5 mm to about 8.0 mm, or from about 0.5 mm to about 4.0 mm, or from about 0.5 mm to about 3.0 mm, or from about 0.5 mm to about 2.0 mm, or from about 0.7 mm to about 1.5 mm.

The presently disclosed subject matter provides for improved nonwoven materials with many advantages over various commercially available materials. For example, the nonwoven materials can be treated with a tacky additive and used on a surface without leaving residue. Additionally, the nonwoven materials can have improved cleaning performance. For example, the nonwoven materials are suitable for attracting and collecting particles. In certain embodiments, the cleaning efficiency of the nonwoven materials can be analyzed by determining the percentage of a mess that is cleaned using the nonwoven materials. In certain embodiments, the nonwoven materials can have a cleaning efficiency that is greater than 10%, greater than 20%, greater than 30%, greater than 40%, greater than 50%, greater than 55%, greater than 60%, greater than 65%, greater than 70%, or greater than 75%. In certain embodiments, the cleaning efficiency of the nonwoven materials can be greater than that of a commercially available product.

In certain embodiments, the cleaning efficiency of the nonwoven materials can be greater for particles having a particular size. For example, in certain embodiments, the nonwoven materials can have a cleaning efficiency that is greater than 40%, greater than 50%, greater than 55%, greater than 60%, greater than 65%, greater than 70%, or greater than 75% for particles having an average particle size of from about 0.35 to about 1.0 mm, or from about 0.4 mm to about 0.8 mm, or from about 0.45 mm to about 0.7 mm, or about 0.5 mm to about 0.6 mm.

In certain embodiments, the nonwoven materials can have a certain tackiness. For example and not limitation, the nonwoven materials can have a tack, *i.e.,* the amount of force needed to separate a tacky portion of the nonwoven material from a surface, of at least about 65 g, at least about 60 g, at least about 100 g, at least about 120 g, at least about 300 g, at least about 500 g, at least about 700 g, at least about 740 g, or at least about 790 g. For further example and not limitation, the nonwoven materials can have a maximum load, *i.e.,* the maximum load that can be sustained between a tacky portion of the nonwoven material and a surface, of at least about 0.68 N (0.15 1bf), at least about 0.77 N (0.17 1bf), at least about 1.11 N (0.25 lbf), at least about 1.20 N (0.27 1bf), at least about 2.22 N (0.5 1bf), at least about 4.45 N (1.0 1bf), at least about 6.23 N (1.4 1bf), at least about 7.12 N (1.6 1bf), or at least about 7.78 N (1.75 lbf).

### Methods of Making the Material

A variety of processes can be used to assemble the materials used in the practice of this disclosed subject matter to produce the materials, including but not limited to, traditional dry forming processes such as airlaying and carding or other forming technologies such as spunlace or airlace. Preferably, the materials can be prepared by airlaid processes. Airlaid processes include, but are not limited to, the use of one or more forming heads to deposit raw materials of differing compositions in selected order in the manufacturing process to produce a product with distinct strata. This allows great versatility in the variety of products which can be produced. In one embodiment, the material is prepared as a continuous airlaid web. The airlaid web is typically prepared by disintegrating or defiberizing a cellulose pulp sheet or sheets, typically by hammermill, to provide individualized fibers. Rather than a pulp sheet of virgin fiber, the hammermills or other disintegrators can be fed with recycled airlaid edge trimmings and off-specification transitional material produced during grade changes and other airlaid production waste. Being able to thereby recycle production waste would contribute to improved economics for the overall process. The individualized fibers from whichever source, virgin or recycled, are then air conveyed to forming heads on the airlaid web-forming machine. A number of manufacturers make airlaid web forming machines suitable for use in the disclosed subject matter, including Dan-Web Forming of Aarhus, Denmark, M&J Fibretech A/S of Horsens, Denmark, Rando Machine Corporation, Macedon, N.Y. which is described in U.S. Pat. No. 3,972,092, the disclosure of which is hereby incorporated by reference in its entirety, Margasa Textile Machinery of Cerdanyola del Valles, Spain, and DOA International of Wels, Austria. While these many forming machines differ in how the fiber is opened and air-conveyed to the forming wire, they all are capable of producing the webs of the presently disclosed subject matter. The Dan-Web forming heads include rotating or agitated perforated drums, which serve to maintain fiber separation until the fibers are pulled by vacuum onto a foraminous forming conveyor or forming wire. In the M&J machine, the forming head is basically a rotary agitator above a screen. The rotary agitator may comprise a series or cluster of rotating propellers or fan blades. Other fibers, such as a synthetic thermoplastic fiber, are opened, weighed, and mixed in a fiber dosing system such as a textile feeder supplied by Laroche S. A. of Cours-La Ville, France. From the textile feeder, the fibers are air conveyed to the forming heads of the airlaid machine where they are further mixed with the comminuted cellulose pulp fibers from the hammer mills and deposited on the continuously moving forming wire. Where defined layers are desired, separate forming heads may be used for each type of fiber. Alternatively or additionally, one or more layers can be prefabricated prior to being combined with additional layers, if any.

The airlaid web is transferred from the forming wire to a calendar or other densification stage to densify the web, if necessary, to increase its strength and control web thickness. In one embodiment, the fibers of the web are then bonded by passage through an oven set to a temperature high enough to fuse the included thermoplastic or other binder materials. In a further embodiment, secondary binding from the drying or curing of a latex spray or foam application occurs in the same oven. The oven can be a conventional through-air oven, be operated as a convection oven, or may achieve the necessary heating by infrared or even microwave irradiation. In particular embodiments, the airlaid web can be treated with additional additives, such as a tacky additive or a binder, before or after heat curing. The additives can be applied by spraying, coating, or printing onto the airlaid web.

### Applications and End Uses

The nonwoven materials of the disclosed subject matter can be used for a variety of applications known in the art. For example, the nonwoven materials can be used either alone or as a component in a variety of consumer products, including cleaning products. In certain aspects, the nonwoven materials can be used as wipes, sheets, towels and the like. By way of example, the nonwoven materials can be used as a disposable wipe for cleaning applications, including household, personal, and industrial cleaning applications. Additionally, the nonwoven materials can be attached to a cleaning tool (*e.g.,* a handed), such as a mop or duster, for cleaning applications.

### 7. EXAMPLES

The following examples are merely illustrative of the presently disclosed subject matter and they should not be considered as limiting the scope of the subject matter in any way.

### EXAMPLE 1: Cleaning efficiency of the nonwoven materials on various surfaces

The present Example provides the cleaning efficiency of two-layer nonwoven materials with and without a tacky additive on various types of surfaces.

The top layer of the two-layer nonwoven material was composed of eccentric bicomponent fibers having a polypropylene core and a polyethylene sheath. The bottom layer was composed of a 12 gsm untreated polypropylene spunbond (scrim code = MOR-B0137) from Polymer Group Inc. Two types of the nonwoven material were prepared to have an overall basis weights of 30 gsm and 50 gsm. Additionally, some samples of each basis weight nonwoven material were treated with a tacky additive on the outer surface of the top layer. The tacky additive was a water based polymer emulsion from Cattie Adhesives (55% solids, 500cps, sprayable viscosity). The target amount of tacky additive was from about 7 gsm to about 8 gsm.

Three different mess formulations were prepared. A first mess formulation (Mess 1) was designed to mimic household debris, and included dirt and hair. A second mess formulation (Mess 2) was designed to mimic fine particulates, and included flour. A third mess formulation (Mess 3) was designed to have a mixture of larger sized particles. Table 1 provides details on each mess formulation.

**Table 1. Mess formulations**

| *Ingredient* | *Mess Recipe Loading Level (g)* | *Notes* |
|---|---|---|
| ***Mess 1*** | | |
| Top Soil | 1.597 | River Run^{®} Top Soil |
| Cat Hair | 0.097 | Shed hair obtained from brushing a short haired double coated domestic cat |
| Salt | 4.632 | Roundy's^{®} Iodized Salt |

| ***Mess 2*** | | |
|---|---|---|
| Flour | 5.376 | Roundy's^{®} All-Purpose Flour |
| Coffee | 0.684 | Maxwell House^{®} Coffee: Original Roast |
| Rice | 0.574 | Roundy's^{®} Medium Grain Rice |

| ***Mess 3*** | | |
|---|---|---|
| Bread Crumbs | 1.202 | Progresso^{®} Panko Bread Crumbs: Plain and Cheerios^{®} |
| Cereal | 0.796 | Cheerios^{®} |
| Cat Food | 0.436 | Alley Cat^{™}: Ocean Fish & Tuna Flavors |
| Coffee | 0.684 | Maxwell House^{®} Coffee: Original Roast |

The messes were weighed and sprinkled evenly onto three different 3 foot x 3 foot floor surfaces (hardwood, vinyl, and ceramic) under a test frame holding a Swiffer mop head such that the Swiffer mop head was suspended over the floor surface and movable across the floor surface upon actuation. The test frame was programed to push the mop head in a set configuration with a constant applied force and speed. The set configuration was designed to cover the entire 3 foot x 3 foot floor surface by moving in a back and forth, snake-like pattern. For consistency, the test frame was used to measure cleaning efficiency for each sample when used in the same pattern, and at the same force and speed, to clean a mess.

Each of the nonwoven materials (*i.e.,* 30 gsm and 50 gsm, and with and without tacky additive) was loaded onto the Swiffer mop head and used to clean each of the mess formulations from a hardwood surface. Additionally, two commercially available materials (Swiffer and 3M Easy Trap Duster) were tested for each mess formulation on the hardwood surface. Each of the nonwoven materials and commercially available materials were tested for three trials. The cleaning efficiency was quantified by calculating the percentage of mess removed by the various materials. In particular, the cleaning efficiency was calculated by first weighing the amount of mess applied, as well as the various materials prior to cleaning. After cleaning, the materials were re-weighed and the original weight was subtracted to calculate the amount of mess picked up by the materials. The cleaning efficiency is the percentage of mess picked up or removed compared to the original amount of mess applied. Table 2 provides the average cleaning efficiency (Avg) and standard deviation (SD) for each material with respect to each mess formulation.

**Table 2. Cleaning efficiency on a hardwood surface**

| | Mess 1 | | Mess 2 | | Mess 3 | |
|---|---|---|---|---|---|---|
| | Avg | SD | Avg | SD | Avg | SD |
| Swiffer | 17.10% | 0.99% | 10.89% | 0.43% | 5.67% | 3.38% |
| 3M | 42.55% | 3.26% | 11.66% | 1.13% | 8.73% | 3.32% |
| 30 gsm | 5.86% | 0.13% | 16.91% | 0.29% | 2.03% | 0.37% |
| 30 gsm + Additive | 55.73% | 0.28% | 19.79% | 4.37% | 14.23% | 2.01% |
| 50 gsm | 5.79% | 0.33% | 9.14% | 2.45% | 1.77% | 0.29% |
| 50 gsm + Additive | 58.82% | 1.87% | 13.97% | 1.96% | 13.15% | 2.05% |

Figure 1 provides an illustratration of the average cleaning efficiency for each of the materials on the hardwood surface. As shown in Table 2 and Figure 1, the nonwoven materials with the tacky additive outperformed the nonwoven materials without the tacky additive across all mess formulations. The nonwoven materials with the tacky additive had improved cleaning efficiency compared to the commercially available materials. Additionally, the nonwoven materials without the tacky additive had improved cleaning efficiency compared to commercially available materials when used to clean Mess 2.

Similarly, each of the nonwoven materials was used to clean each mess formulation from a vinyl surface. The Swiffer and 3M materials were also tested by cleaning each mess formulation from the vinyl surface. Each of the nonwoven materials and commercially available materials were tested for three trials. Table 3 provides the average cleaning efficiency and standard deviation for each material with respect to each mess formulation.

**Table 3. Cleaning efficiency on a vinyl surface**

| | Mess 1 | | Mess 2 | | Mess 3 | |
|---|---|---|---|---|---|---|
| | Avg | SD | Avg | SD | Avg | SD |
| Swiffer | 23.45% | 0.90% | 29.58% | 4.55% | 5.79% | 0.72% |
| 3M | 44.51% | 5.41% | 29.67% | 1.47% | 9.37% | 3.41% |
| 30 gsm | 4.44% | 0.41% | 22.09% | 1.67% | 4.64% | 2.06% |
| 30 gsm + Additve | 64.91% | 2.38% | 32.24% | 4.59% | 16.65% | 7.36% |
| 50 gsm | 5.50% | 0.41% | 33.39% | 2.93% | 3.59% | 0.65% |
| 50 gsm + Additive | 68.61% | 2.72% | 32.20% | 0.53% | 20.82% | 3.82% |

Figure 2 provides an illustratration of the average cleaning efficiency for each of the materials on the vinyl surface. As with the hardwood surface, the nonwoven materials with the tacky additive outperformed the nonwoven materials without the tacky additive across all mess formulations. The nonwoven materials with the tacky additive had improved cleaning efficiency compared to the commercially available materials across all mess formulations. Additionally, the 50 gsm nonwoven material without the tacky additive had improved cleaning efficiency compared to the commercially available materials when used to clean Mess 2.

Furthermore, each of the nonwoven materials was used to clean each mess formulation from a ceramic surface. The Swiffer and 3M materials were also tested by cleaning each mess formulation from the ceramic surface. Each of the nonwoven materials and commercially available materials were tested for three trials. Table 4 provides the average cleaning efficiency and standard deviation for each material with respect to each mess formulation.

**Table 4. Cleaning efficiency on a ceramic surface**

| | Mess 1 | | Mess 2 | | Mess 3 | |
|---|---|---|---|---|---|---|
| | Avg | SD | Avg | SD | Avg | SD |
| Swiffer | 30.84% | 0.33% | 59.55% | 3.22% | 24.87% | 3.97% |
| 3M | 70.29% | 0.00% | 39.86% | 5.03% | 16.18% | 2.08% |
| 30 gsm | 4.97% | 0.54% | 28.16% | 0.46% | 5.20% | 0.63% |
| 30 gsm + Additive | 44.75% | 3.06% | 38.19% | 4.87% | 23.39% | 3.47% |
| 50 gsm | 7.54% | 0.86% | 37.92% | 1.06% | 5.13% | 1.06% |
| 50 gsm + Additive | 59.83% | 4.62% | 41.25% | 1.13% | 17.75% | 5.45% |

Figure 3 provides an illustratration of the average cleaning efficiency for each of the materials on the ceramic surface. As with the hardwood and vinyl surfaces, the nonwoven materials with the tacky additive outperformed the nonwoven materials without the tacky additive across all mess formulations. The nonwoven materials with the tacky additive had improved cleaning efficiency compared to the commercially available materials when used to clean Mess 1. Furthermore, compared to the Swiffer product, both the 30 gsm and the 50 gsm nonwoven materials with the tacky additive had improved cleaning efficiency when used to clean Mess 1. Compared to the 3M product, the 30 gsm nonwoven material with the tacky additive had improved cleaning efficiency when used to clean Mess 3.

These data show that the nonwoven materials of the presently disclosed subject matter can be more effective for cleaning purposes than certain commercially available materials. Furthermore, the addition of a tacky additive can significantly increase the cleaning efficiency of a nonwoven material. Particularly, applying a tacky additive to an outer bicomponent layer of a nonwoven material can improve its cleaning performance.

### EXAMPLE 2: Cleaning efficiency of nonwoven materials with respect to particle sizes

The present Example provides the cleaning efficiency of an experimental nonwoven material. The top layer of the nonwoven material was composed of eccentric bicomponent fibers having a polypropylene core and a polyethylene sheath. The bottom layer was composed of a 12 gsm untreated polypropylene spunbond (scrim code = MOR-B0137) from Polymer Group Inc. The nonwoven material had an overall basis weight of 50 gsm. The outer surface of the top layer was treated with a tacky additive. The tacky additive was a water based polymer emulsion from Cattie Adhesives (55% solids, 500cps, sprayable viscosity). The target amount of tacky additive was from about 7 gsm to about 8 gsm.

Four mess formulations (Cheerios, Cat Food, Bread Crumbs, and Cat Litter) were prepared by crushing and sieving the formulations into specific particle sizes. Each of the mess formulations was separated in groups by particle size (based on diameter). The particle size groups were: (1) greater than 6.3 mm (">6.3 mm"); (2) from 3.15 mm to 6.3 mm ("3.15-6.3 mm"); (3) from 1.6 mm to 3.15 mm ("1.6-3.15 mm"); and (4) less than 1.6 mm ("<1.6 mm"). Each particle size group was applied to three different surfaces (hardwood, vinyl, and ceramic), and cleaned using the nonwoven material as described in Example 1. Each nonwoven material was weighed prior to loading onto the mop head and after cleaning to determine the weight of the mess removed. For comparison, each particle size group was also cleaned using a commercially available material (Swiffer). Three trials were performed for each particle size group.

The cleaning efficiency was quantified by calculating the percentage of mess removed by the various materials. In the Tables and Figures below, "Tacky" refers to the nonwoven material of this Example. Table 5 provides the average cleaning efficiency and standard deviation of the nonwoven material and the Swiffer material with respect to each mess formulation on a hardwood surface.

**Table 5. Cleaning efficiency on a hardwood surface**

| | | **Tacky Nonwoven** | | **Swiffer** | |
|---|---|---|---|---|---|
| **Mess Formulation** | | Avg | SD | Avg | SD |
| *Cheerios* | | | | | |
| | >6.3mm | 8.92% | 14.32% | 3.99% | 5.55% |
| | 3.15-6.3mm | 3.68% | 3.79% | 9.05% | 2.20% |
| | 1.6-3.15mm | 36.03% | 5.83% | 8.64% | 1.85% |
| | <1.6mm | 49.12% | 7.45% | 29.80% | 5.82% |
| *Cat Food* | | | | | |
| | >6.3mm | 2.20% | 3.61% | 0.78% | 0.96% |
| | 3.15-6.3mm | 3.93% | 4.67% | 0.52% | 0.56% |
| | 1.6-3.15mm | 20.60% | 9.11% | 3.98% | 1.56% |
| | <1.6mm | 59.81% | 6.47% | 32.04% | 14.98% |
| *Bread Crumbs* | | | | | |
| | >6.3mm | 16.18% | 13.90% | 7.26% | 6.38% |
| | 3.15-6.3mm | 9.50% | 6.71% | 4.86% | 1.08% |
| | 1.6-3.15mm | 37.80% | 4.12% | 11.45% | 2.65% |
| | <1.6mm | 75.69% | 5.40% | 32.39% | 6.34% |
| Cat Litter | | | | | |
| | 1.6-3.15mm | 53.91% | 9.25% | 1.21% | 1.08% |
| | <1.6mm | 67.24% | 5.27% | 4.23% | 1.10% |

Figures 4A-4D provide an illustration of the average cleaning efficiency ("pickup effectiveness") of each material for each particle size group. For most messes, the nonwoven material showed improved cleaning efficiency compared to the Swiffer material. Particularly, the nonwoven material had significantly greater cleaning efficiency for small particles sizes (*e.g.,* 1.6-3.15 mm and < 1.6 mm).

Table 6 provides the average cleaning efficiency and standard deviation of the nonwoven material and the Swiffer material with respect to each formulation on a vinyl surface.

**Table 6. Cleaning efficiency on a vinyl surface**

| | | **Tacky Nonwoven** | | **Swiffer** | |
|---|---|---|---|---|---|
| **Mess Formulation** | | Avg | SD | Avg | SD |
| *Cheerios* | | | | | |
| | >6.3mm | 6.38% | 7.42% | 0.08% | 0.37% |
| | 3.15-6.3mm | 5.99% | 3.41% | 8.24% | 10.96% |
| | 1.6-3.15mm | 38.33% | 5.61% | 10.17% | 2.09% |
| | <1.6mm | 69.36% | 1.69% | 50.55% | 0.54% |
| *Cat Food* | | | | | |
| | >6.3mm | 0.54% | 0.63% | 0.00% | 0.00% |
| | 3.15-6.3mm | 5.73% | 8.06% | -0.30% | 0.34% |
| | 1.6-3.15mm | 14.41% | 7.88% | 4.94% | 1.80% |
| | <1.6mm | 64.07% | 6.35% | 43.22% | 2.72% |
| *Bread Crumbs* | | | | | |
| | >6.3mm | 7.13% | 2.51% | 2.31% | 1.69% |
| | 3.15-6.3mm | 16.22% | 3.38% | 1.59% | 1.01% |
| | 1.6-3.15mm | 50.28% | 5.58% | 7.14% | 4.08% |
| | <1.6mm | 67.64% | 10.68% | 49.80% | 4.11% |
| Cat Litter | | | | | |
| | 1.6-3.15mm | 41.06% | 4.33% | 0.92% | 0.05% |
| | <1.6mm | 62.49% | 1.35% | 6.26% | 0.76% |

Figures 5A-5D provide an illustration of the average cleaning efficiency ("pickup effectiveness") of each material on a vinyl surface for each particle size group. As with the hardwood surface, the nonwoven material showed improved cleaning efficiency compared to the Swiffer material. The nonwoven material had significantly greater cleaning efficiency across all particles sizes, particularly with respect to the Cat Food, Bread Crumbs, and Cat Litter mess formulations.

Table 7 provides the average cleaning efficiency and standard deviation of the nonwoven material and the Swiffer material with respect to each formulation on a ceramic surface.

**Table 7. Cleaning efficiency on a ceramic surface**

| | | **Tacky Nonwoven** | | **Swiffer** | |
|---|---|---|---|---|---|
| **Mess Formulation** | | Avg | SD | Avg | SD |
| | *Cheerios* | | | | |
| | >6.3mm | 9.65% | 4.93% | 16.67% | 9.17% |
| | 3.15-6.3mm | 19.17% | 9.48% | 39.50% | 10.96% |
| | 1.6-3.15mm | 37.90% | 3.93% | 53.77% | 2.45% |
| | <1.6mm | 57.43% | 1.84% | 74.90% | 2.26% |
| *Cat Food* | | | | | |
| | >6.3mm | 1.46% | 0.70% | 32.62% | 54.92% |
| | 3.15-6.3mm | 13.25% | 6.65% | 13.20% | 8.86% |
| | 1.6-3.15mm | 23.23% | 6.36% | 34.00% | 2.41% |
| | <1.6mm | 53.62% | 5.72% | 69.75% | 0.79% |
| *Bread Crumbs* | | | | | |
| | >6.3mm | 4.03% | 2.33% | 20.63% | 6.68% |
| | 3.15-6.3mm | 24.66% | 6.46% | 47.58% | 34.50% |
| | 1.6-3.15mm | 35.34% | 2.77% | 55.73% | 7.01% |
| | <1.6mm | 58.10% | 9.12% | 75.03% | 1.41% |
| Cat Litter | | | | | |
| | 1.6-3.15mm | 20.01% | 9.10% | 1.22% | 0.03% |
| | <1.6mm | 63.23% | 0.41% | 14.28% | 2.85% |

Figures 6A-6D provide an illustration of the average cleaning efficiency ("pickup effectiveness") of each material on a ceramic surface for each particle size group. The nonwoven material had significantly greater cleaning efficiency compared to the Swiffer material with respect to the Cat Litter. The Cat Litter contained only small particles (*e.g.,* 1.6-3.15 mm and < 1.6 mm), suggesting that the nonwoven material has improved cleaning performance with respect to fine particles.

These data show that the nonwoven materials of the presently disclosed subject matter have improved cleaning efficiency over certain commercially available materials. Particularly, treating an outer layer of a nonwoven material with a tacky additive can provide improved cleaning performance.

### EXAMPLE 3: Cleaning efficiency of nonwoven materials with respect to soil type

The present Example provides the cleaning efficiency of nonwoven materials in cleaning messes comprising various soil types.

A first sample (Sample 3) was a two-layer nonwoven material having an overall basis weight of 50 gsm. The top layer of the two-layer nonwoven material was composed of eccentric bicomponent fibers having a polypropylene core and a polyethylene sheath. The bottom layer was composed of a 12 gsm untreated polypropylene spunbond (scrim code = MOR-B0137) from Polymer Group Inc. The nonwoven material was treated with a tacky additive on the outer surface of the top layer. The tacky additive was a water based polymer emulsion from Cattie Adhesives (55% solids, 500cps, sprayable viscosity). The target amount of tacky additive was about 1 gsm, *i.e.,* from about 0.8 gsm to about 1.3 gsm. For comparison purpose, the two-layer nonwoven material (Sample 3) was compared to commercially available Swiffer and Great Value products.

Eight different mess formulations were prepared, each containing a single soil type. The soil types were: coffee, crushed Cheerios, cat litter, salt, sand, sugar, and flour, which was tested in twice on two different days. The sources of each soil type are listed in Table 8, below. The messes were weighed and sprinkled evenly onto a 3 foot x 3 foot vinyl floor surfaces under a test frame holding a Swiffer mop head. The test frame was programed to push the mop head in a set configuration with a constant applied force and speed, as described in Example 1.

**Table 8. Soil types**

| | |
|---|---|
| Cat Litter | Roundy's^{®} Pet Care Kitty Litter |
| Coffee | Maxwell House^{®} Medium Coffee: Original Roast |
| Crushed Cheerios | Cheerios^{®} crushed with Cobb test roller |
| Flour | Roundy's^{®} All-Purpose Flour |
| Salt | Roundy's^{®} Table Salt |
| Sand | Quikrete^{®} All-Purpose Sand |
| Sugar | Crystal Sugar Granulated White Sugar (American Crystal Sugar Company) |

Sample 3 and the Swiffer product were loaded onto the Swiffer mop head and used to clean each of the mess formulations from the vinyl surface. Each of the nonwoven materials and commercially available materials were tested for three trials with each mess formulation, except that the Swiffer product was only tested with flour (flour mess 2) for one trial. Additionally, the Great Value product was only tested with flour (flour mess 2), salt, cat litter, and coffee. The cleaning efficiency was quantified by calculating the percentage of mess removed by the various materials. In particular, the cleaning efficiency was calculated by first weighing the amount of mess applied, as well as the various materials prior to cleaning. After cleaning, the materials were re-weighed and the original weight was subtracted to calculate the amount of mess picked up by the materials. The cleaning efficiency is the percentage of mess picked up or removed compared to the original amount of mess applied. Table 9 provides the average cleaning efficiency (Avg) and standard deviation (SD) for each material with respect to each mess formulation.

**Table 9. Cleaning efficiency (%)**

| | Sample 3 | | Swiffer | | Great Value | |
|---|---|---|---|---|---|---|
| | Avg | SD | Avg | SD | Avg | SD |
| Cat Litter | 49.86 | 1.24 | 12.17 | 0.69 | 4.06 | 0.75 |
| Coffee | 24.13 | 0.37 | 23.57 | 1.93 | 23.53 | 2.62 |
| Crushed Cheerios | 22.61 | 1.83 | 21.83 | 0.94 | N/A | N/A |
| Flour (1) | 48.08 | 10.54 | 41.63 | 2.30 | N/A | N/A |
| Flour (2) | 35.65 | 1.99 | 35.30 | N/A | 33.15 | 1.57 |
| Salt | 60.68 | 1.38 | 32.91 | 0.14 | 8.61 | 2.37 |
| Sand | 45.84 | 2.47 | 21.72 | 1.50 | N/A | N/A |
| Sugar | 53.48 | 1.86 | 32.47 | 0.86 | N/A | N/A |

Figure 7A depicts the average cleaning efficiency of Sample 3 as compared to the Swiffer product across each of the soil types and Figure 7B depicts the average cleaning efficiency of Sample 3 as compared to the Great Value product across each of the soil types tested for the Great Value product. For further illustration, Figure 8 provides a bar chart of the cleaning efficiencies of Sample 3, the Swiffer product, and the Great Value product for the four soil types tested with each product: cat litter, coffee, flour, and salt.

These data show that Sample 3 had a cleaning efficiency that was at least as good as that of the Swiffer and Great Value products for each soil type. For example, as compared to the Swiffer product, Sample 3 had approximately the same cleaning efficiency for flour, coffee, and Cheerios. As compared to the Great Value product, Sample 3 had approximately the same cleaning efficiency for flour and coffee.

However, for several of the soil types, Sample 3 had significantly improved cleaning efficiency as compared to the Swiffer and Great Value products. For example, as compared to the Swiffer product, Sample 3 had twice (2x) the cleaning efficiency for salt, sugar, and sand. As compared to the Great Value product, Sample 3 had seven times (7x) the cleaning efficiency for salt. Sample 3 performed much better than both the Swiffer and Great Value products for cat litter. Sample 3 had four times (4x) the cleaning efficiency of the Swiffer product and twelve times (12x) the cleaning efficiency of the Great Value product when used to clean cat litter.

To further analyze the relationship between soil type and cleaning efficiency, the particle size distributions for several soil types were determined, and are provided in Figure 9 for sugar, salt, sand, crushed Cheerios, and cat litter. The average particle sizes for these soil types ranged from about 0.38 mm (for sugar) to about 1 mm (for crushed Cheerios). Figures 10A-10B provide the cleaning efficiencies as compared to particle size for each of Sample 3 and the Swiffer product, respectively. As shown in Figures 10A-10B, cleaning efficiency was generally related to particle size. For example, Sample 3 had the best cleaning efficiency for medium-sized particles having an average particle size of about 0.5 mm (*see* Figure 10A, salt) as compared to the other sized particles tested. However, the Swiffer product generally had worse cleaning efficiency across all particle sizes and did not have a peak cleaning efficiency for for medium-sized particles having an average particle size of about 0.5 mm, as compared to smaller-sized particles (*see* Figure 10B, sugar and salt).

As such, these data indicate that, nonwoven materials according to the presently disclosed subject matter can have improved cleaning efficiency as compared to commercially available alternatives. For example, the presently disclosed nonwoven materials can have particularly improved cleaning efficiency for certain particle sizes and soil types.

### EXAMPLE 4: Tack testing of nonwoven materials

The present Example measures the tackiness of nonwoven materials prepared in accordance with the presently disclosed subject matter.

Four sample nonwoven materials were prepared (Samples 4A-4D). Samples 4B-4D were treated with a tacky additive on the outer surface of their top layer. For Sample 4B, the target amount of tacky additive was about 1 gsm. For Samples 4C and 4D, the target amount of tacky additive was about 6 gsm. Sample 4C was prepared from a 6.25 gsm nonwoven material whereas Sample 4D was prepared from a 6.77 gsm nonwoven material. Sample 4A was not treated with a tacky additive.

To measure the tackiness of Samples 4A-4D, the samples were taped to a 2 inch x 2 inch upper platform on a tensile frame. The samples were lowered to be adjacent a bottom platform, until the pressure between the platforms reached 0.425 psi. The platform was subsequently raised and the load between the upper and bottom platforms was measured. Table 10 below provides the average maximum load and tack for four trials of each sample. The maximum load is the peak load between the upper and bottom platforms, whereas the tack is the force needed to separate the two platforms.

**Table 10. Average maximum load and tack of Samples 4A-4D**

| | Max Load (N) [lbf] | Tack (g) |
|---|---|---|
| Sample 4A | 0.2740 [0.0616] | 28.0 |
| Sample 4B | 1.2036 [0.2706] | 122.9 |
| Sample 4C | 7.7871 [1.7506] | 794.8 |
| Sample 4D | 7.2817 [1.6370] | 743.2 |

As illustrated in Table 10, the nonwoven materials including a tacky additive (Samples 4B-4D) had significantly greater maximum load and tack as compared to those without a tacky additive. Additionally, the samples were greater amounts of tacky additive (Samples 4C and 4C) has the greatest maximum load and tack.

To further investigate the tackiness of the presently disclosed nonwoven materials, three additional samples of nonwoven materials were prepared (Samples 4E-4G). Samples 4E and 4F were prepared on two different converters, but each contained the same amount of tacky additive. Sample 4G was prepared on the same converter as Sample 4F, but did not contain any tacky additive. Tackiness was tested according to the methods described above. Table 11 below provides the maximum load and tack for four trials of each sample.

**Table 11. Average maximum load and tack of Samples 4E-4G**

| | Max Load [lbf] | Tack (g) |
|---|---|---|
| Sample 4E | 0.7620 [0.1713] | 77.8 |
| Sample 4F | 0.6837 [0.1537] | 69.8 |
| Sample 4G | 0.2535 [0.0570] | 25.9 |

As illustrated in Table 11, regardless of the converter used, the nonwoven materials including a tacky additive (Samples 4E and 4F) had significantly more tackiness, as measured by maximum load and tack, as compared to Sample 4G without a tacky additive. However, the conversion process did impact tackiness, as Sample 4F had less tackiness than Sample 4E.

These data illustrate that a tacky additive can be used to impart tackiness to the presently disclosed nonwoven materials. When treated with the tacky additive, the nonwoven materials can be used to pick up and sustain high loads, and would be suitable for collecting and trapping soils, as described above.

## Claims

1. A multi-layer nonwoven material, comprising:
a first outer layer comprising synthetic fibers and a tacky additive; and
a second outer layer comprising cellulose fibers and/or synthetic fibers,
wherein, when used to clean a surface, the nonwoven material has a cleaning efficiency of at least about 20%; and
wherein the nonwoven material has a tack of at least about 65 g.

2. A multi-layer nonwoven material, comprising:
an outer layer comprising synthetic fibers and a tacky additive; and
an absorbent core,
wherein, when used to clean a surface, the nonwoven material has a cleaning efficiency of at least about 20%; and
wherein the nonwoven material has a tack of at least about 65 g.

3. The multi-layer nonwoven material of claim 2, wherein the absorbent core comprises:
a first layer comprising cellulose fibers;
a second layer, adjacent to the first layer, comprising SAP;
a third layer, adjacent to the second layer, comprising cellulose fibers;
a fourth layer, adjacent to the third layer, comprising SAP; and
a fifth layer, adjacent to the fourth layer, comprising cellulose fibers.

4. The multi-layer nonwoven material of claim 1 or of claim 2, wherein the nonwoven material has a cleaning efficiency of at least about 35% or of at least about 50%.

5. The multi-layer nonwoven material of claim 1 or of claim 2, wherein the nonwoven material has a cleaning efficiency of at least 60% with respect to particles having an average particle size of from about 0.4 mm to about 1.0 mm, and optionally or preferably, wherein the nonwoven material has a cleaning efficiency of at least 60% with respect to particles having an average particle size of from about 0.5 mm to about 0.6 mm.

6. The multi-layer nonwoven material of claim 1 or of claim 2, wherein the nonwoven material has a tack of:
(i) at least about 120 g; or
(ii) at least about 790 g.

7. The multi-layer nonwoven material of claim 1 or of claim 2, wherein the nonwoven material has a maximum load of
(i) at least about 0.68 N (0.15 lbf); or
(ii) at least about 1.11 N (0.25 lbf).
(iii) at least about 7.78 N (1.75 lbf).

8. The multi-layer nonwoven material of claim 1, wherein the synthetic fibers of the first outer layer are bicomponent fibers.

9. The multi-layer nonwoven material of claim 1, wherein the second outer layer comprises synthetic fibers.

10. The multi-layer nonwoven material of claim 1 or of claim 2, wherein the tacky additive is present on at least a portion of an external surface of the first outer layer; or
wherein the tacky additive is a pressure sensitive adhesive.

11. The multi-layer nonwoven material of claim 1 or of claim 2, wherein the tacky additive is present in an amount of:
(i) from about 0.5 gsm to about 10 gsm; or
(ii) from about 7 gsm to about 8 gsm; or
(iii) from about 0.5 gsm to about 1.5 gsm.

12. The multi-layer nonwoven material of claim 1, wherein at least one of the first outer layer and the second outer layer comprises binder.

13. The multi-layer nonwoven material of claim 1, wherein the first outer layer comprises bicomponent fibers and cellulose fibers; or
wherein the second outer layer comprises cellulose fibers and synthetic fibers.

14. The multi-layer nonwoven material of claim 1, further comprising an intermediate layer comprising bicomponent fibers.

15. A cleaning wipe, comprising the multi-layer nonwoven material of any one of claims 1-14.

16. A mop head, comprising the multi-layer nonwoven material of any one of claims 1-15.

## Patentansprüche

1. Mehrschichtiges Vliesmaterial, umfassend:
eine erste äußere Schicht, die synthetische Fasern und ein klebriges Additiv umfasst; und
eine zweite äußere Schicht, die Cellulosefasern und/oder synthetische Fasern umfasst,
wobei das Vliesmaterial, wenn es zum Reinigen einer Oberfläche verwendet wird, eine Reinigungswirkung von mindestens etwa 20% aufweist; und
wobei das Vliesmaterial eine Klebrigkeit von mindestens etwa 65 g aufweist.

2. Mehrschichtiges Vliesmaterial, umfassend:
eine äußere Schicht, die synthetische Fasern und ein klebriges Additiv umfasst; und
einen absorbierenden Kern,
wobei das Vliesmaterial, wenn es zum Reinigen einer Oberfläche verwendet wird, eine Reinigungswirkung von mindestens etwa 20% aufweist; und
wobei das Vliesmaterial eine Klebrigkeit von mindestens etwa 65 g aufweist.

3. Das mehrschichtige Vliesmaterial nach Anspruch 2, wobei der absorbierende Kern umfasst:
eine erste Schicht, die Cellulosefasern umfasst;
eine zweite Schicht, die an die erste Schicht angrenzt und SAP umfasst; und
eine dritte Schicht, die an die zweite Schicht angrenzt und Cellulosefasern umfasst;
eine vierte Schicht, die an die dritte Schicht angrenzt und SAP umfasst; und
eine fünfte Schicht, die an die vierte Schicht angrenzt und Cellulosefasern umfasst.

4. Das mehrschichtige Vliesmaterial nach Anspruch 1 oder Anspruch 2, wobei das Vliesmaterial eine Reinigungswirkung von mindestens etwa 35 % oder von mindestens etwa 50 % aufweist.

5. Das mehrschichtige Vliesmaterial nach Anspruch 1 oder 2, wobei das Vliesmaterial eine Reinigungswirkung von mindestens 60 % in Bezug auf Partikel mit einer durchschnittlichen Partikelgröße von etwa 0,4 mm bis etwa 1,0 mm aufweist, und optional oder vorzugsweise, wobei das Vliesmaterial eine Reinigungswirkung von mindestens 60 % in Bezug auf Partikel mit einer durchschnittlichen Partikelgröße von etwa 0,5 mm bis etwa 0,6 mm aufweist.

6. Das mehrschichtige Vliesmaterial nach Anspruch 1 oder Anspruch 2, wobei das Vliesmaterial eine Klebrigkeit aufweist von:
(i) mindestens etwa 120 g; oder
(ii) mindestens etwa 790 g.

7. Das mehrschichtige Vliesmaterial nach Anspruch 1 oder Anspruch 2, wobei das Vliesmaterial eine maximale Belastung aufweist von
(i) mindestens etwa 0,68 N (0,15 lbf);
(ii) mindestens etwa 1,11 N (0,25 lbf); oder
(iii) mindestens etwa 7,78 N (1,75 lbf).

8. Das mehrschichtige Vliesmaterial nach Anspruch 1, wobei die synthetischen Fasern der ersten äußeren Schicht Bikomponentenfasern sind.

9. Das mehrschichtige Vliesmaterial nach Anspruch 1, wobei die zweite äußere Schicht synthetische Fasern umfasst.

10. Das mehrschichtige Vliesmaterial nach Anspruch 1 oder Anspruch 2, wobei das klebrige Additiv auf mindestens einem Teil einer Außenfläche der ersten äußeren Schicht vorhanden ist; oder
wobei das klebrige Additiv ein Haftkleber ist.

11. Das mehrschichtige Vliesmaterial nach Anspruch 1 oder Anspruch 2, wobei das klebrige Additiv vorhanden ist in einer Menge von:
(i) von etwa 0,5 g/m² bis etwa 10 g/m²; oder
(ii) von etwa 7 g/m² bis etwa 8 g/m²; oder
(iii) von etwa 0,5 g/m² bis etwa 1,5 g/m².

12. Das mehrschichtige Vliesmaterial nach Anspruch 1, wobei mindestens eine der ersten äußeren Schicht und der zweiten äußeren Schicht ein Bindemittel enthält.

13. Das mehrschichtige Vliesmaterial nach Anspruch 1, wobei die erste äußere Schicht Bikomponentenfasern und Cellulosefasern umfasst; oder
wobei die zweite äußere Schicht Cellulosefasern und synthetische Fasern umfasst.

14. Das mehrschichtige Vliesmaterial nach Anspruch 1, das ferner eine Zwischenschicht umfasst, die Bikomponentenfasern umfasst.

15. Reinigungstuch, umfassend das mehrschichtige Vliesmaterial nach einem der Ansprüche 1-14.

16. Moppkopf, umfassend das mehrschichtige Vliesmaterial nach einem der Ansprüche 1-15.

## Revendications

1. Matériau multicouche non-tissé comprenant :
une première couche externe comprenant des fibres synthétiques et un additif collant, et
une seconde couche externe comprenant des fibres de cellulose et/ou des fibres synthétiques,
dans lequel, lorsqu'il est utilisé pour nettoyer une surface, le matériau non-tissé présente un rendement de nettoyage d'au moins environ 20 %, et
où le matériau non-tissé présente une pégosité d'au moins environ 65 g.

2. Matériau multicouche non-tissé comprenant :
une couche externe comprenant des fibres synthétiques et un additif collant, et
une âme absorbante,
dans lequel, lorsqu'il est utilisé pour nettoyer surface, le matériau non-tissé présente un rendement de nettoyage d'au moins environ 20 %, et
où le matériau non-tissé présente une pégosité d'au moins environ 65 g.

3. Matériau multicouche non-tissé selon la revendication 2, dans lequel l'âme absorbante comprend :
une première couche comprenant des fibres de cellulose,
une deuxième couche, adjacente à la première couche, comprenant du SAP,
une troisième couche, adjacente à la deuxième couche, comprenant des fibres de cellulose,
une quatrième couche, adjacente à la troisième couche, comprenant du SAP, et
une cinquième couche, adjacente à la quatrième couche, comprenant des fibres de cellulose.

4. Matériau multicouche non-tissé selon la revendication 1 ou la revendication 2, le matériau non-tissé présentant un rendement de nettoyage d'au moins environ 35 % ou d'au moins environ 50 %.

5. Matériau multicouche non-tissé selon la revendication 1 ou la revendication 2, le matériau non-tissé présentant un rendement de nettoyage d'au moins 60 % par rapport à des particules présentant une taille moyenne de particules depuis environ 0,4 mm jusqu'à environ 1,0 mm, et en option ou de préférence, où le matériau non-tissé présente un rendement de nettoyage d'au moins 60 % par rapport à des particules présentant une taille moyenne de particules depuis environ 0,5 mm jusqu'à environ 0,6 mm.

6. Matériau multicouche non-tissé selon la revendication 1 ou la revendication 2, le matériau non-tissé présentant une pégosité de :
(i) au moins environ 120 g, ou
(ii) au moins environ 790 g.

7. Matériau multicouche non-tissé selon la revendication 1 ou la revendication 2, le matériau non-tissé présentant une charge maximale de :
(i) au moins environ 0,68 N (0,15 lbf), ou
(ii) au moins environ 1,11 N (0,25 lbf),
(iii) au moins environ 7,78 N (1,75 lbf).

8. Matériau multicouche non-tissé selon la revendication 1, dans lequel les fibres synthétiques de la première couche externe sont des fibres bi composant.

9. Matériau multicouche non-tissé selon la revendication 1, dans lequel la seconde couche externe comprend des fibres synthétiques.

10. Matériau multicouche non-tissé selon la revendication 1 ou la revendication 2, dans lequel l'additif collant est présent sur au moins une partie d'une surface externe de la première couche externe, ou
dans lequel l'additif collant et un adhésif sensible à la pression.

11. Matériau multicouche non-tissé selon la revendication 1 ou la revendication 2, dans lequel l'additif collant est présent en une teneur de :
(i) d'environ 0,5 g/m² à environ 10 g/m², ou
(ii) d'environ 7 g/m² à environ 8 g/m²,
(iii) d'environ 0,5 g/m² à environ 1,5 g/m².

12. Matériau multicouche non-tissé selon la revendication 1, dans lequel au moins l'une de la première couche externe et de la seconde couche externe comprend un liant.

13. Matériau multicouche non-tissé selon la revendication 1, dans lequel la première couche externe comprend des fibres bi composant et des fibres de cellulose, ou
dans lequel la seconde couche externe comprend des fibres de cellulose et des fibres synthétiques.

14. Matériau multicouche non-tissé selon la revendication 1, comprenant en outre une couche intermédiaire comprenant des fibres bi composant.

15. Chiffon de nettoyage comprenant le matériau multicouche non-tissé conforme à l'une quelconque des revendications 1 à 14.

16. Tête de balai à laver comprenant le matériau multicouche non-tissé conforme à l'une quelconque des revendications 1 à 15.
